# EUROPÄISCHE PATENTANMELDUNG

(11) **EP 1 178 053 A2**
(43) Veröffentlichungstag der Anmeldung: **06.02.2002**
(21) Anmeldenummer: 01118709.3
(22) Anmeldetag: 03.08.2001
(51) Int. Cl.: C07K 14/72, C12N 15/12, C07K 19/00, C12N 15/62, C12N 5/10, C12Q 1/68, A61K 38/17, A61K 48/00, A61K 39/395, A61P 17/02, A61P 17/06

(54) **Polypeptide oder diese kodierende Nukleinsäuren einer Familie von G-Protein gekoppelten Rezeptoren sowie deren Verwendung zur Diagnose oder Behandlung von Hautkrankheiten**

(30) Priorität: 04.08.2000 DE 10038111; 31.08.2000 US 229501 P
(71) Anmelder: Switch Biotech Aktiengesellschaft, 82152 Martinsried (DE)
(72) Erfinder: Wolf, Eckhard, Prof. Dr., 85764 Oberschleissheim (DE); Werner, Sabine, Prof. Dr., 8032 Zürich (CH); Halle, Jörn-Peter, Dr., 82337 Penzberg (DE); Regenbogen, Johannes, Dr., 82152 Martinsried (DE); Goppelt, Andreas, Dr., 80636 München (DE)
(74) Vertreter: Bösl, Raphael, Dr. rer. nat., Dipl.-Chem.

(57) **Zusammenfassung**

Die Erfindung betrifft Polypeptide oder diese kodierende Nukleinsäuren einer Familie von G-Protein-gekoppelten Rezeptoren sowie deren Verwendung zur Diagnose, Prävention und/oder Behandlung von Erkrankungen, beispielsweise Hauterkrankungen, und/oder Diagnose und/oder Behandlung bei der Wundheilung und/oder deren krankhaften Störungen, und/oder zur Identifizierung von pharmakologisch aktiven Substanzen.

Sequenzprotokoll

<110> SWITCH Biotech AG

<120> Polypeptide oder diese kodierende Nukleinsäuren einer Familie von G-
      Protein gekoppelten Rezeptoren sowie deren Verwendung zur Diagnose
      oder Behandlung von Erkrankungen, beispielsweise Hautkrankheiten,
      sowie ihre Verwendung zur Identifizierung von pharmakologisch aktiven
      Substanzen

<130> S34321EP

<160> 21

<170> WORD6.0, PC-DOS/MS-DOS

<210> 1
<211> 331
<212> PRT
<213> Mus musculus

<400> 1
Met Gly Glu Ser Asn Gly Glu Ala Phe Leu Ala Phe Lys Thr Ser Ala
  1               5                  10                  15

Ser Pro Thr Ala Pro Val Thr Thr Asn Pro Met Asp Glu Thr Leu Pro
             20                  25                  30

Gly Ser Ile Asn Ile Arg Ile Leu Ile Pro Lys Leu Met Ile Ile Ile
         35                  40                  45

Phe Gly Leu Val Gly Leu Met Gly Asn Ala Ile Val Phe Trp Leu Leu
     50                  55                  60

Gly Phe His Leu Arg Arg Asn Ala Phe Ser Val Tyr Ile Leu Asn Leu
 65                  70                  75                  80

Ala Leu Ala Asp Phe Leu Phe Leu Leu Ser Ser Ile Ile Ala Ser Thr

85                  90                  95

Leu Phe Leu Leu Lys Val Ser Tyr Leu Ser Ile Ile Phe His Leu Cys
            100                 105                 110

Phe Asn Thr Ile Met Met Val Val Tyr Ile Thr Gly Ile Ser Met Leu
        115                 120                 125

Ser Ala Ile Ser Thr Glu Cys Cys Leu Ser Val Leu Cys Pro Thr Trp
    130                 135                 140

Tyr Arg Cys His Arg Pro Val His Thr Ser Thr Val Met Cys Ala Val
145                 150                 155                 160

Ile Trp Val Leu Ser Leu Leu Ile Cys Ile Leu Asn Ser Tyr Phe Cys
                165                 170                 175

Ala Val Leu His Thr Arg Tyr Asp Asn Asp Asn Glu Cys Leu Ala Thr
            180                 185                 190

Asn Ile Phe Thr Ala Ser Tyr Met Ile Phe Leu Leu Val Val Leu Cys
        195                 200                 205

Leu Ser Ser Leu Ala Leu Leu Ala Arg Leu Phe Cys Gly Ala Gly Gln
    210                 215                 220

Met Lys Leu Thr Arg Phe His Val Thr Ile Leu Leu Thr Leu Leu Val
225                 230                 235                 240

Phe Leu Leu Cys Gly Leu Pro Phe Val Ile Tyr Cys Ile Leu Leu Phe
                245                 250                 255

Lys Ile Lys Asp Asp Phe His Val Leu Asp Val Asn Leu Tyr Leu Ala
            260                 265                 270

Leu Glu Val Leu Thr Ala Ile Asn Ser Cys Ala Asn Pro Ile Ile Tyr

        275                 280                 285

Phe Phe Val Gly Ser Phe Arg His Gln Leu Lys His Gln Thr Leu Lys
    290                 295                 300

Met Val Leu Gln Ser Ala Leu Gln Asp Thr Pro Glu Thr Ala Glu Asn
305                 310                 315                 320

Met Val Glu Met Ser Ser Asn Lys Ala Glu Pro

<210> 2
<211> 321
<212> PRT
<213> Homo sapiens

<400> 2
Met Asn Gln Thr Leu Asn Ser Ser Gly Thr Val Glu Ser Ala Leu Asn
1               5                   10                  15

Tyr Ser Arg Gly Ser Thr Val His Thr Ala Tyr Leu Val Leu Ser Ser
            20                  25                  30

Leu Ala Met Phe Thr Cys Leu Cys Gly Met Ala Gly Asn Ser Met Val
        35                  40                  45

Ile Trp Leu Leu Gly Phe Arg Met His Arg Asn Pro Phe Cys Ile Tyr
    50                  55                  60

Ile Leu Asn Leu Ala Ala Ala Asp Leu Leu Phe Leu Phe Ser Met Ala
65                  70                  75                  80

Ser Thr Leu Ser Leu Glu Thr Gln Pro Leu Val Asn Thr Thr Asp Lys
                85                  90                  95

Val His Glu Leu Met Lys Arg Leu Met Tyr Phe Ala Tyr Thr Val Gly

            100                 105                 110

Leu Ser Leu Leu Thr Ala Ile Ser Thr Gln Arg Cys Leu Ser Val Leu
        115                 120                 125

Phe Pro Ile Trp Phe Lys Cys His Arg Pro Arg His Leu Ser Ala Trp
    130                 135                 140

Val Cys Gly Leu Leu Trp Thr Leu Cys Leu Leu Met Asn Gly Leu Thr
145                 150                 155                 160

Ser Ser Phe Cys Ser Lys Phe Leu Lys Phe Asn Glu Asp Arg Cys Phe
                165                 170                 175

Arg Val Asp Met Val Gln Ala Ala Leu Ile Met Gly Val Leu Thr Pro
            180                 185                 190

Val Met Thr Leu Ser Ser Leu Thr Leu Phe Val Trp Val Arg Arg Ser
        195                 200                 205

Ser Gln Gln Trp Arg Arg Gln Pro Thr Arg Leu Phe Val Val Val Leu
    210                 215                 220

Ala Ser Val Leu Val Phe Leu Ile Cys Ser Leu Pro Leu Ser Ile Tyr
225                 230                 235                 240

Trp Phe Val Leu Tyr Trp Leu Ser Leu Pro Pro Glu Met Gln Val Leu
                245                 250                 255

Cys Phe Ser Leu Ser Arg Leu Ser Ser Ser Val Ser Ser Ser Ala Asn
            260                 265                 270

Pro Val Ile Tyr Phe Leu Val Gly Ser Arg Arg Ser His Arg Leu Pro
        275                 280                 285

Thr Arg Ser Leu Gly Thr Val Leu Gln Gln Ala Leu Arg Glu Glu Pro

290                 295                 300

Glu Leu Glu Gly Gly Glu Thr Pro Thr Val Gly Thr Asn Glu Met Gly
305                 310                 315                 320

Ala

<210> 3
<211> 325
<212> PRT
<213> Mus musculus

<400> 3
Met Asp Ile Asp Ile Ser Ser Leu Gly Ile Tyr Ile Ile Ala Pro Asn
1               5                   10                  15

Gly Ser Ser Tyr Thr Asn Ser Val Asp Cys Phe Phe Lys Ile Gln Val
            20                  25                  30

Met Gly Phe Leu Ser Leu Ile Ile Ser Pro Val Gly Met Val Leu Asn
        35                  40                  45

Ser Thr Val Leu Trp Phe Leu Gly Phe Gln Ile Arg Arg Asn Ala Phe
    50                  55                  60

Ser Val Tyr Ile Leu Asn Leu Ala Gly Ala Asp Phe Leu Phe Leu His
65                  70                  75                  80

Ser Gln Phe Leu Phe Tyr Leu Leu Ala Ile Phe Pro Ser Ile Pro Ile
                85                  90                  95

Gln Ile Pro Leu Phe Phe Asp Met Leu Thr Lys Phe Ala Tyr Leu Ser
            100                 105                 110

Gly Leu Ser Ile Leu Ser Thr Ile Ser Ile Glu Arg Cys Leu Cys Val
        115                 120                 125

Met Trp Pro Ile Trp Tyr Arg Cys Gln Arg Pro Arg His Thr Ser Ser
    130                 135                 140

Val Thr Cys Ser Leu Leu Trp Ala Leu Ser Leu Leu Phe Ala Leu Leu
145                 150                 155                 160

Asp Gly Met Gly Cys Gly Leu Leu Phe Asn Ser Phe Asp Gln Ser Trp
                165                 170                 175

Cys Leu Lys Phe Asp Leu Ile Ile Cys Ala Trp Ser Ile Val Leu Phe
            180                 185                 190

Val Val Leu Cys Gly Ser Ser Leu Ile Leu Leu Val Arg Ile Phe Cys
        195                 200                 205

Gly Ser Gln Gln Ile Pro Val Thr Arg Leu Tyr Val Thr Ile Ala Leu
    210                 215                 220

Thr Val Leu Phe Phe Leu Ile Cys Cys Leu Pro Phe Gly Ile Ser Trp
225                 230                 235                 240

Ile Ile Gln Trp Ser Glu Thr Leu Ile Tyr Val Gly Phe Cys Asp Tyr
                245                 250                 255

Phe His Glu Glu Leu Phe Leu Ser Cys Ile Asn Ser Cys Ala Asn Pro
            260                 265                 270

Ile Ile Tyr Phe Leu Val Gly Phe Ile Arg Gln Arg Lys Phe Gln Gln
        275                 280                 285

Lys Ser Leu Lys Val Leu Leu Gln Arg Ala Met Glu Asp Thr Pro Glu
    290                 295                 300

Glu Glu Asn Glu Asp Met Gly Pro Ser Arg Asn Pro Glu Glu Phe Glu
305                 310                 315                 320

Thr Val Cys Ser Asn
                325

<210> 4
<211> 330
<212> PRT
<213> Homo sapiens

<400> 4
Met Asp Pro Thr Thr Pro Ala Trp Gly Thr Glu Ser Thr Thr Val Asn
  1               5                  10                  15

Gly Asn Asp Gln Ala Leu Leu Leu Leu Cys Gly Lys Glu Thr Leu Ile
             20                  25                  30

Pro Val Phe Leu Ile Leu Phe Ile Ala Leu Val Gly Leu Val Gly Asn
         35                  40                  45

Gly Phe Val Leu Trp Leu Leu Gly Phe Arg Met Arg Arg Asn Ala Phe
     50                  55                  60

Ser Val Tyr Val Leu Ser Leu Ala Gly Ala Asp Phe Leu Phe Leu Cys
 65                  70                  75                  80

Phe Gln Ile Ile Asn Cys Leu Val Tyr Leu Ser Asn Phe Phe Cys Ser
                 85                  90                  95

Ile Ser Ile Asn Phe Pro Ser Phe Phe Thr Thr Val Met Thr Cys Ala
            100                 105                 110

Tyr Leu Ala Gly Leu Ser Met Leu Ser Thr Val Ser Thr Glu Arg Cys
        115                 120                 125

Leu Ser Val Leu Trp Pro Ile Trp Tyr Arg Cys Arg Arg Pro Arg His
    130                 135                 140

Leu Ser Ala Val Val Cys Val Leu Leu Trp Ala Leu Ser Leu Leu Leu
145                 150                 155                 160

Ser Ile Leu Glu Gly Lys Phe Cys Gly Phe Leu Phe Ser Asp Gly Asp
                165                 170                 175

Ser Gly Trp Cys Gln Thr Phe Asp Phe Ile Thr Ala Ala Trp Leu Ile
            180                 185                 190

Phe Leu Phe Met Val Leu Cys Gly Ser Ser Leu Ala Leu Leu Val Arg
        195                 200                 205

Ile Leu Cys Gly Ser Arg Gly Leu Pro Leu Thr Arg Leu Tyr Leu Thr
    210                 215                 220

Ile Leu Leu Thr Val Leu Val Phe Leu Leu Cys Gly Leu Pro Phe Gly
225                 230                 235                 240

Ile Gln Trp Phe Leu Ile Leu Trp Ile Trp Lys Asp Ser Asp Val Leu
                245                 250                 255

Phe Cys His Ile His Pro Val Ser Val Val Leu Ser Ser Leu Asn Ser
            260                 265                 270

Ser Ala Asn Pro Ile Ile Tyr Phe Phe Val Gly Ser Phe Arg Lys Gln
        275                 280                 285

Trp Arg Leu Gln Gln Pro Ile Leu Lys Leu Ala Leu Gln Arg Ala Leu
    290                 295                 300

Gln Asp Ile Ala Glu Val Asp His Ser Glu Gly Cys Phe Arg Gln Gly
305                 310                 315                 320

Thr Pro Glu Met Ser Arg Ser Ser Leu Val
                325                 330

<210> 5
<211> 993
<212> DNA
<213> Mus musculus

<400> 5
atgggggaaa gcaatggtga agcatttctt gcctttaaga cctcagcctc accaacagca 60
ccagtgacaa caaatccaat ggacgaaacc ctccctggaa gtatcaacat taggattctg 120
atcccaaaat tgatgatcat catcttcgga ctggtcggac tgatgggaaa cgccattgtg 180
ttctggctcc tgggcttcca cttgcgcagg aatgccttct cagtctacat cctaaacttg 240
gccctggctg acttcctttt cctcctcagt agtatcatag cttccaccct gtttcttctc 300
aaagtttcct acctcagcat catctttcac ttgtgcttta acaccattat gatggttgtc 360
tacatcacag ggataagcat gctcagtgcc atcagcactg agtgctgcct gtctgtcctg 420
tgccccacct ggtatcgctg ccaccgtcca gtacatacat caactgtcat gtgtgctgtg 480
atctgggtcc tatccctgtt gatctgcatt ctgaatagct atttctgtgc tgtcttacat 540
accagatatg ataatgacaa tgagtgtctg gcaactaaca tctttaccgc ctcgtacatg 600
atatttttgc ttgtggtcct ctgtctgtcc agcctggctc tgctggccag gttgttctgt 660
ggcgctgggc agatgaagct taccagattt catgtgacca tcttgctgac ccttttggtt 720
tttctcctct gcgggttgcc ctttgtcatc tactgcatcc tgttattcaa gattaaggat 780
gatttccatg tattagatgt taatctttat ctagcattag aagtcctgac tgctattaac 840
agctgtgcca accccatcat ctacttcttc gtgggctctt tcagacatca gttgaagcac 900
cagaccctca aaatggttct ccagagtgca ctgcaggaca ctcctgagac agctgaaaac 960
atggtagaga tgtcaagtaa caaagcagag cct 993

<210> 6
<211> 966
<212> DNA
<213> Homo sapiens

<400> 6

atgaaccaga ctttgaatag cagtgggacc gtggagtcag ccctaaacta ttccagaggg 60

agcacagtgc acacggccta cctggtgctg agctccctgg ccatgttcac ctgcctgtgc 120

gggatggcag gcaacagcat ggtgatctgg ctgctgggct ttcgaatgca caggaacccc 180

ttctgcatct atatcctcaa cctggcggca gccgacctcc tcttcctctt cagcatggct 240

tccacgctca gcctggaaac ccagcccctg gtcaatacca ctgacaaggt ccacgagctg 300

atgaagagac tgatgtactt tgcctacaca gtgggcctga gcctgctgac ggccatcagc 360

acccagcgct gtctctctgt cctcttccct atctggttca agtgtcaccg gcccaggcac 420

ctgtcagcct gggtgtgtgg cctgctgtgg acactctgtc tcctgatgaa cgggttgacc 480

tcttccttct gcagcaagtt cttgaaattc aatgaagatc ggtgcttcag ggtggacatg 540

gtccaggccg ccctcatcat gggggtctta accccagtga tgactctgtc cagcctgacc 600

ctctttgtct gggtgcggag gagctcccag cagtggcggc ggcagcccac acggctgttc 660

gtggtggtcc tggcctctgt cctggtgttc ctcatctgtt ccctgcctct gagcatctac 720

tggtttgtgc tctactggtt gagcctgccg cccgagatgc aggtcctgtg cttcagcttg 780

tcacgcctct cctcgtccgt aagcagcagc gccaaccccg tcatctactt cctggtgggc 840

agccggagga gccacaggct gcccaccagg tccctgggga ctgtgctcca acaggcgctt 900

cgcgaggagc ccgagctgga aggtggggag acgcccaccg tgggcaccaa tgagatgggg 960

gcttga 966

<210> 7

<211> 978

<212> DNA

<213> Mus musculus

<400> 7

atggatatag acatttcgag cctgggcatt tacatcatag caccgaatgg aagcagctac 60

actaatagtg ttgattgttt cttcaaaatc caagtcatgg gttttctttc cctcatcatt 120

tcccctgttg ggatggtatt aaattccaca gtgctgtggt ttctgggctt ccagatacgt 180

aggaatgcct tctctgtcta catcctcaac ctggccgggg ctgactttct cttcctgcac 240

tctcagtttt tattttacct tcttgctatt tttccctcca ttcctatcca gatccctctc 300

ttttttgata tgttgacaaa atttgcatat ctttctgggc tgagcattct cagcaccatt 360

agcattgagc gctgcctgtg tgtcatgtgg cccatctggt accgctgtca aagaccaaga 420

cacacatcat ctgtaacctg ttccttgctt tgggctttgt ccctgttgtt tgctcttctg 480

gatgggatgg gatgtggctt actgtttaat agttttgacc agtcttggtg tttgaaattt 540

gatttaatca tttgtgcgtg gtcaattgtt ttatttgtgg ttctctgtgg gtccagtctc 600

atcctacttg ttaggatctt ctgtggctcc cagcagatcc ctgtgaccag gctgtatgtg 660

accattgcac tcacagtgtt attcttccta atctgctgtc ttccctttgg aatctcctgg 720
atcatccaat ggagtgaaac tttgatatat gttggatttt gtgattattt tcacgaggaa 780
ctattcctat cctgtattaa cagctgtgcc aaccctatca tttacttcct tgttggtttt 840
attcgtcagc gaaagttcca acagaagtct ctgaaggtgc ttcttcaaag agcgatggag 900
gacactcctg aagaagaaaa tgaagacatg ggtccttcaa gaaatccaga agaatttgaa 960
acagtctgta gcaactga 978

<210> 8
<211> 1770
<212> DNA
<213> Homo sapiens

<400> 8
aatcagtgaa catgcagcag gtgctcaagt cttgtttttg tttccagggg caccagtgga 60
ggttttctga gcatggatcc aaccaccccg gcctggggaa cagaaagtac aacagtgaat 120
ggaaatgacc aagcccttct tctgctttgt ggcaaggaga ccctgatccc ggtcttcctg 180
atccttttca ttgccctggt cgggctggta ggaaacgggt ttgtgctctg gctcctgggc 240
ttccgcatgc gcaggaacgc cttctctgtc tacgtcctca gcctggccgg ggccgacttc 300
ctcttcctct gcttccagat tataaattgc ctggtgtacc tcagtaactt cttctgttcc 360
atctccatca atttccctag cttcttcacc actgtgatga cctgtgccta ccttgcaggc 420
ctgagcatgc tgagcaccgt cagcaccgag cgctgcctgt ccgtcctgtg gcccatctgg 480
tatcgctgcc gccgccccag acacctgtca gcggtcgtgt gtgtcctgct ctgggccctg 540
tccctactgc tgagcatctt ggaagggaag ttctgtggct tcttatttag tgatggtgac 600
tctggttggt gtcagacatt tgatttcatc actgcagcgt ggctgatttt tttattcatg 660
gttctctgtg ggtccagtct ggccctgctg gtcaggatcc tctgtggctc caggggtctg 720
ccactgacca ggctgtacct gaccatcctg ctcacagtgc tggtgttcct cctctgcggc 780
ctgccctttg gcattcagtg gttcctaata ttatggatct ggaaggattc tgatgtctta 840
ttttgtcata ttcatccagt ttcagttgtc ctgtcatctc ttaacagcag tgccaacccc 900
atcatttact tcttcgtggg ctcttttagg aagcagtggc ggctgcagca gccgatcctc 960
aagctggctc tccagagggc tctgcaggac attgctgagg tggatcacag tgaaggatgc 1020
ttccgtcagg gcaccccgga gatgtcgaga agcagtctgg tgtagagatg gacagcctct 1080
acttccatca gatatatgtg gctttgagag gcaactttgc ccctgtctgt ctgatttgct 1140
gaactttctc agtcctgatt ttaaaacagt taagagagtc cttgtgagga ttaagtgaga 1200
cagtgcctat gaaacaaaca ctaagtgcag tgtctctgga actgccttac tcacaggctt 1260
ccaccacagc cctatgagag ctttgccaac tctgcggtcc atgactgttc ccacttttaa 1320

tgaatcctac ctttcgcaga aggctgaaag cagggcagaa aagatctaca tttctttgga 1380
cactgcactt gatagggact caaagaatgt tatattttta attaatttct ttttctcttc 1440
cgtacaattt ctgtctcaac aaaattagaa gaattaaatt taaaactagc tccaaaagag 1500
cagccgtctt tcattttggc agaccttaga atatccccct agcttaataa atctttgttg 1560
aatggcttaa tgaatgaata aactggttaa tgtttaagtt aaacctctga aaagtctcca 1620
tttaccagat ttgagtcact aaatttattg ctttcactac ttttgaattc acctctccgt 1680
tatgacccta aatcacaatc tccattctga ctgttgatgg actcctctca gtcattaagg 1740
gtggccatgg gtcatggcct gagtcatttc 1770

<210> 9
<211> 24
<212> DNA
<213> Mus musculus

<400> 9
gaggcatgtc aaatcagtaa gctg 24

<210> 10
<211> 20
<212> DNA
<213> Mus musculus

<400> 10
ggtggctttg gagtgagcac 20

<210> 11
<211> 19
<212> DNA
<213> Mus musculus

<400> 11
atcaacggga agcccatca 19

<210> 12

<211> 20

<212> DNA

<213> Mus musculus

<400> 12

gacatactca gcaccggcct                                                    20

<210> 13

<211> 21

<212> DNA

<213> Homo sapiens

<400> 13

ttcttctgct ttgtggcaag g                                                  21

<210> 14

<211> 21

<212> DNA

<213> Homo sapiens

<400> 14

gaaaaggatc aggaagaccg g                                                  21

<210> 15

<211> 653

<212> DNA

<213> Mus musculus

<400> 15

tattcaggag gggtagaaaa actaagttag tggagagatg ttgaagttgt tggtggacca 60

ctgaggcatg tcaaatcagt aagctgagag aagagtggac agtggtcaag tgcagcaggg 120

cagtgctcac tccaaagcca cctctgaggt ccaggtagag gctcttcatc aaggctctgc 180
tttgttactt gacatctcta ccatgttttc agctgtctca ggagtgtcct gcagtgcact 240
ctggagaacc attttgaggg tctggtgctt caactgatgt ctgaaagagc ccacgaagaa 300
gtagatgatg gggttggcac agctgttaat agcagtcagg acttctaatg ctagataaag 360
attaacatct aatacatgga aatcatcctt aatcttgaat aacaggatgc agtagatgac 420
aaagggcacc cgcagaggag aaaaaccaaa agggtcanca agatgggcac atgaaatctg 480
gnaagcttta tntgccccag cgccacaaaa acaacctggc canaaaaaac cngngntggn 540
cangacnggg nncccncccn ccaaaanttt tttttntttn ctgnccnggg gnggnccttt 600
tnnaaagccc atntttccna ccacccctng ggngggggcc nttttttttt ggg 653

<210> 16
<211> 22
<212> DNA
<213> Homo sapiens

<400> 16
catgggtgtg aaccatgaga ag 22

<210> 17
<211> 21
<212> DNA
<213> Homo sapiens

<400> 17
ctaagcagtt ggtggtgcag g 21

<210> 18
<211> 25
<212> DNA
<213> Homo sapiens

<400> 18
TCTTAACCAC CAGATCATTC CTTCT 25

<210> 19

<211> 20

<212> DNA

<213> Homo sapiens

<400> 19
GGATACTGCG AGCAAATGGG 20

<210> 20

<211> 24

<212> DNA

<213> Homo sapiens

<400> 20

GGAGTCAGCC CTAAACTATT CCAG 24

<210> 21

<211> 20

<212> DNA

<213> Homo sapiens

<400> 21

AGGTAGGCCG TGTGCACTGT 20

## Beschreibung

Die Erfindung betrifft Polypeptide oder diese kodierende Nukleinsäuren einer Familie von G-Protein gekoppelten Rezeptoren sowie deren Verwendung zur Diagnose, Prävention und/oder Behandlung von Erkrankungen, beispielsweise Hauterkrankungen, und/oder Diagnose und/oder Behandlung bei der Wundheilung und/oder deren krankhaften Störungen und/oder zur Identifizierung von pharmakologisch aktiven Substanzen.

Wunden heilen im allgemeinen ohne therapeutischen Eingriff ab. Es gibt jedoch zahlreiche Erkrankungen, bei denen die Wundheilung eine Rolle spielt, wie z.B. Diabetes mellitus, arterielle Verschlußkrankheiten, Schuppenflechte (Psoriasis), Morbus Crohn, Epidermolysis bullosa, altersbedingte Hautveränderungen oder Innervationsstörungen. Wundheilungsstörungen führen zu einer verzögerten Wundheilung oder zu chronischen Wunden. Diese Störungen können durch die Art der Verwundung (z.B. großflächige Wunden, tiefgehende und mechanisch gedehnte Operationswunden, Verbrennungen, Trauma, Dekubitus), medikamentöse Behandlung der Patienten (z.B. mit Corticoiden) aber auch durch die Art der Erkrankung selber verursacht werden. So leiden z.B. 25% der Patienten mit Typ-II-Diabetes häufig an chronischen Ulzera ("diabetischer Fuß"), von denen etwa die Hälfte aufwendige stationäre Behandlungen erfordern und letztlich doch schlecht heilen. Der diabetische Fuß verursacht mehr Klinikaufenthalte als jede andere mit Diabetes assoziierte Komplikation. Die Zahl dieser Fälle bei Diabetes Typ I und II ist im Steigen begriffen und repräsentiert 2,5% aller Klinikeinweisungen. Zudem heilen Wunden mit zunehmendem Alter der Patienten schlechter. Oft ist auch eine Beschleunigung des natürlichen Wundheilungsprozesses wünschenswert, um z.B. die Gefahr von bakteriellen Infektionen oder die Liegezeiten der Patienten zu verringern.

Auch nach erfolgtem Wundverschluß kann es zu weiteren Störungen kommen. Während Wunden fötaler Haut ohne Narbenbildung heilen, kommt es nach Verletzungen in der Postnatalperiode stets zu Narbenbildungen, die oft ein großes kosmetisches Problem darstellen. Bei Patienten mit großflächigen Brandwunden kann zudem die Lebensqualität dramatisch beeinträchtigt werden, zumal vernarbter Haut auch die Anhangsgebilde, wie Haarfollikel, Schweiß- und Talgdrüsen fehlen. Bei entsprechender genetischer Disposition kann es auch zu Keloiden kommen, hypertrophischen Narben, die in die umgebende Haut einwuchern.

Der Vorgang der Wundheilung erfordert komplexe koordiniert ablaufende Wirkungen und Interaktionen verschiedener Zelltypen. Im Wundheilungsprozeß unterscheidet man folgende Schritte: die Blutgerinnung im Bereich der Wunde, die Rekrutierung von Entzündungszellen, die Reepithelialisierung, die Bildung von Granulationsgewebe sowie die Restrukturierung von Matrix. Die exakten Reaktionsmuster der beteiligten Zelltypen während der Phasen der Proliferation, der Migration, der Matrixsynthese und der Kontraktion sind, ebenso wie die Regulation von Genen wie z.B. Wachstumsfaktoren, Rezeptoren und Matrixproteinen, bislang wenig bekannt.

So sind bisher nur wenig zufriedenstellende Therapien entwickelt worden, um bei Wundheilungsstörungen eingreifen zu können. Etablierte Therapieformen beschränken sich auf physikalische Unterstützung der Wundheilung (z.B. Verbände, Kompressen, Gele) oder der Transplantation von Hautgeweben, gezüchteten Hautzellen und/oder Matrixproteinen. In den letzten Jahren sind Wachstumsfaktoren zur Verbesserung der Wundheilung erprobt worden, ohne jedoch die konventionelle Therapie entscheidend zu verbessern. Auch die Diagnose von Wundheilungsstörungen beruht auf wenig aussagekräftigen optischen Analysen der Haut, da ein tieferes Verständnis der Genregulation während der Wundheilung bisher fehlt.

Auch für andere Störungen von regenerativen Prozessen sind bisher wenig zufriedenstellende Therapien entwickelt worden. Auch hier ist die Kenntnis der Genregulation vorteilhaft für die Entwicklung von Diagnostika und Therapien. Es ist gezeigt worden (Finch et al., 1997, Am. J. Pathol. 151: 1619-28; Werner, 1998, Cytokine Growth Factor Rev. 9: 153-165), daß wundheilungsrelevante Gene auch bei dermatologischen Erkrankungen, die auf Störungen der Regeneration der Haut beruhen, und allgemein bei regenerativen Prozessen eine entscheidende Rolle spielen. So spielt der Wachstumsfaktor KGF nicht nur eine entscheidende Rolle bei der Regulation der Proliferation und Differenzierung von Keratinozyten während der Wundheilung, sondern ist auch ein wichtiger Faktor bei der Hyperproliferation der Keratinozyten bei der Schuppenflechte (Psoriasis) und Regenerationsprozessen im Darm (bei Morbus Crohn und ulcerativer Colitis).

Neue Möglichkeiten bei der Modulation der Wundheilung und der Behandlung von Wundheilungsstörungen können sich durch die Untersuchung von G-Protein gekoppelten Rezeptoren (GPCR) im Zusammenhang mit Wundheilung eröffnen. Die Superfamilie der GPCR, die die größte bislang bekannte Rezeptorfamilie darstellt, ist durch sieben hoch konservierte, charakteristische Sequenzmotive von jeweils 20-30 Aminosäuren Länge gekennzeichnet, die sich durch eine hohe Hydrophobizität auszeichnen (Probst et al., 1992, DNA and Cell Biol., 11: 1-20; Flower, 1999, Biochem. Biophys. Acta, 1422: 207-234). Die Signaltransduktion erfolgt meist über heterotrimere G-Proteine, die wiederum sekundäre Botenstoffe, wie cAMP, cGMP, Diacylglycerol oder Inositol-1,4,5-trisphosphat regulieren (Watson und Arkinstall (Hrsg.), 1999, The G-Protein Linked Receptors Facts Book, Academic Press, New York). Es können jedoch auch MAP Kinasen aktiviert werden (Lefkowitz, 1998, J. Biol. Chem., 273: 18677-18680). Die Stimuli, die auf GPCRs wirken umfassen ein breites Spektrum, das von Licht, Riechstoffen, Neuromodulatoren bis zu verschiedensten Hormonen reicht (Lerner et al., 1993, Ciba Found. Symp., 179: 76-87), was die medizinische Bedeutung der GPCR begründet. Es wird geschätzt, daß über 50% aller modernen Medikamente auf GPCRs wirken (Gudermann et al., 1995, J. Mol. Med. 73: 51-63). Die bislang identifizierten Medikamente konzentrieren sich beispielsweise auf die Modulation des Gonadotropin-Releasing Hormon Rezeptors, wodurch Prostata- und Brust-Karzinome als auch Endometriose und frühzeitige Pubertät behandelt werden können (Pace et al., 1992, Am. Fam. Physician, 44: 1777-1782). Propanolol, ein Antagonist des α-adrenergen Rezeptors im Herz, wird dagegen zur Behandlung von Bluthochdruck, Angina Pektoris und psychischen Störungen eingesetzt (Nace und Wood, 1987, Clin. Pharmacokinet. 13:51-64; Ananth und Lin, 1986, Neuropsychobiology, 15: 20-27). Metaproterenol, ein Antagonist des β2-adrenergen Rezeptors der Lunge, kommt bei der Erweiterung der Bronchien zur Anwendung (Hurst, 1973, Ann. Allergy, 31: 460-466).

Dagegen werden Agonisten oder Antagonisten von GPCRs bei Wundheilungsprozessen selten eingesetzt. Ein Antagonist des Histamin 2 Rezeptors kann beispielsweise eingesetzt werden, um Ulzera und idiopathische Urtikaria zu behandeln (Sontag et al., 1984, N. Engl. J. Med., 311: 689-693; Choy und Middleton, 1991, DICP, 25: 609-612). Zudem wurden "Protease-aktivierte Rezeptor 3 Proteine" (US 5,892,014), Nukleotid-Rezeptoren (WO 98/32429; WO 94/23723), Angiotensin Rezeptoren (WO 98/33813), der CCR-5 Rezeptor (WO 98/30218) und der Thromboxan A2 Rezeptor (US 4,851,413) als mögliche Targets bei der Modulation der Wundheilung und/oder Behandlung von Wundheilungserkrankungen beschrieben. Ferner wurden gegen GPCR der Genfamilie PF4AR (IL-8) gerichtete Antikörper (US 6087475, US 5776457) mit der Diagnose und der Behandlung von Entzündungen der Haut (US 5776457) und Wundheilung (US 6087475) in Verbindung gebracht, wobei sich dies ausschließlich auf experimentellen Daten, die sich auf das Lungengewebe (US 5776457) bzw. auf Charakterisierung der Antikörper an neutrophile Granulozyten (US 6087475) beziehen, stützen. Aus US 6025154 ist ein G-protein Chemokin Rezeptor Polypeptid bekannt, daß unter einer Vielzahl von Krankheiten, die durch Therapie mit G-protein Chemokin Rezeptor Polypeptiden interagierenden Substanzen behandelt werden können, auch Wundheilung nennt. Die in den genannten Schriften (US 6087475, US 5776457, US 6025154) beschriebenen Sequenzen weisen keine signifikante Sequenzidentität zu den erfindungsgemäßen Polypeptiden und Nukleinsäuren der vorliegenden Erfindung auf. Es ist für einen Fachmann keine erfolgversprechende Strategie, von der Verwendbarkeit eines GPCR der einen Genfamilie zur Diagnose oder Therapie von Hauterkrankungen oder Wundheilung auf die Verwendbarkeit eines GPCR einer ganz anderen Genfamilie zur Diagnose oder Behandlung der selben Krankheiten zu schließen, da aufgrund der Diversität der GPCRs und der von Ihnen kontrollierten physiologischen Prozesse keine Vorhersagen zur Funktion der GPCR auf der Grundlage der bloßen Zugehörigkeit zur Superfamilie der G-Protein gekoppelten Rezeptoren möglich sind.

Ausgenommen der olfaktorischen GPCRs, von denen 1000-2000 im Mensch vorhanden sind, wurden bislang mehrere hundert Mitglieder der GPCR-Superfamilie identifiziert (Flower, Biochem. Biophys. Acta, 1999, 1422: 207-234). Über 80 dieser Rezeptoren gehören zu den sogenannten Orphan-Rezeptoren, denen noch kein Ligand zugeordnet werden konnte (Marchese et al., 1999, Trends in Pharmacol. Sci., 20: 370-375). Durch die Identifizierung und Analyse von Orphan-Rezeptoren im Zusammenhang von Erkrankungen eröffnen sich neue Möglichkeiten zur Behandlung dieser Erkrankungen (Marchese et al., supra), zumal eine Vielzahl spezieller Methoden zum Screening von Antagonisten und Agonisten von GPCRs zur Verfügung stehen (Lerner et al., supra; WO 96/41169; US 5,482,835; WO 99/06535; EP 0939902; WO 99/66326; WO 98/34948; EP 0863214; US 5,882,944; US 5,891,646).

Aus WO 99/41364 ist ein Verfahren zur Identifizierung von wundheilungsrelevanten Genen bekannt, das auf der Verwendung von sogenannten Healer-Mäusen beruht. Healer-Mäuse zeichnen sich vor allem durch verbesserte Knorpelheilung aus, und es ist daher zweifelhaft, ob auch die Wundheilung der Haut verbessert ist und ob diese Mäuse daher zur Identifizierung von wundrelevanten und hauterkrankungsrelevanten Genen geeignet sind. Weiterhin ist nicht bekannt, welche Gene mutiert sind; aus den Tabellen 3 und 4 geht hervor, daß eine ganze Reihe von chromosomalen Loci in Healer-Mäusen betroffen sind. Es ist daher fraglich, ob die unter Verwendung dieser Mäuse identifizierten Gene für die Wundheilung oder Hauterkrankungen im Menschen überhaupt von Bedeutung sind, da die differentielle Aktivität der identifizierten Gene lediglich auf Wirkungen der mutierten Gene und nicht auf Vorgänge der Hauterkrankung oder der Wundheilung zurückzuführen sein könnte.

Es ist daher Aufgabe der vorliegenden Erfindung, neue Polypeptide aus der Superfamilie der G-Protein gekoppelten Rezeptoren und diese kodierende Nukleinsäuren zur Verfügung zu stellen, die an Prozessen bei Erkrankungen von Säugetierzellen, beispielsweise bei Erkrankungen von Hautzellen und/oder bei der Wundheilung und/oder deren krankhaften Störungen beteiligt sind, und deren Verwendung die Diagnose, Prävention und/oder Behandlung sowie die Identifizierung und Entwicklung von wirksamen Pharmazeutika entscheidend verbessert.

Bei der Analyse der Genexpression während des Wundheilungsprozesses konnten überraschenderweise bisher unbekannte, zueinander homologe Gene identifiziert werden, die eine Genfamilie innerhalb der Superfamilie der G-Protein gekoppelten Rezeptoren bilden und eine mäßig große Homologie zum Orphan Rezeptor Mas (Young et al., 1986, Cell, 45:711-719) zu den "dorsal root" GPCR Rezeptoren (WO 99/32519) und zum murinen ERG9 GPCR (JP 2000023677) aufweisen (ca. 30-50 % Sequenzidentität zwischen den Polypeptiden). Menschliches SW1695 ist aus WO200119983 als IGS3 GPCR und aus WO200116159 als Ant GPCR bekannt. Dagegen ist murines SW1695 neu und weist beispielsweise zum CanoMan GPCR aus der Maus (WO200116177) eine Identität auf der Aminosäureebene von 53% über einen Bereich von 331 Aminosäuren auf. Die Identität von murinem SW1695 zu IGS3 und Ant auf der Aminosäureebene beträgt 53% über einen Bereich von 314 Aminosäuren. Humanes SW1368 weist über einen Bereich von 285 Aminosäuren eine Identität von 43% zu IGS3 und Ant auf, während die Identität zwischen murinem SW1368 und Ant bzw. IGS3 50 % über einen Bereich von 306 Aminosäuren beträgt.

Die Funktion der IGS3 und Ant Polypeptide oder der diese Polypeptide kodierenden Nukleinsäuren ist nicht bekannt. Während die Herkunft/Isolierung der Ant GPCR Nukleinsäure- und Polypeptidsequenz bis auf den Herkunftsorganismus (Homo sapiens) unbekannt ist, ist bei IGS3 bekannt, daß die Nukleinsäuresequenz durch Überlagerung von partiellen genomischen humanen Sequenzen generiert wurde.

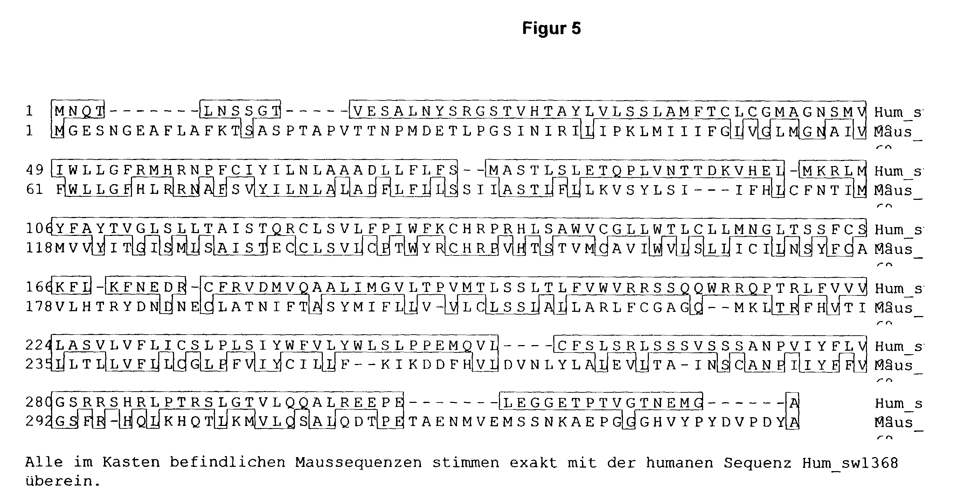

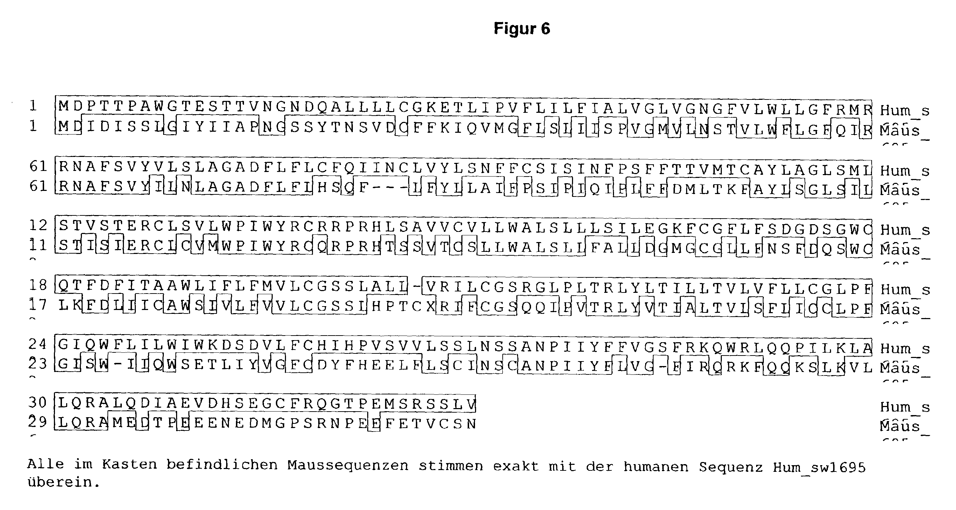

Die neuen Polypeptide dieser Gene sind essentiell für den Wundheilungsprozeß und bieten die Möglichkeit eines neuartigen Therapieansatzes zur Behandlung von Erkrankungen, insbesondere von Erkrankungen der Hautzellen und/oder bei der Wundheilung und/oder deren krankhaften Störungen. Die nicht durch öffentliche Datenbanken zugänglichen Polypeptidsequenzen der erfindungsgemäßen Polypeptide und ihrer cDNAs sind im Sequenzprotokoll aufgelistet (Polypeptidsequenzen: SEQ ID Nr. 1 bis SEQ ID Nr. 4, cDNA-Sequenzen: SEQ ID Nr. 5 bis SEQ ID Nr. 8). Figur 5 und Figur 6 zeigen den Vergleich der menschlichen und murinen Polypeptidsequenzen von SW1368 (SEQ ID Nr. 1 und 2) und SW1695 (SEQ ID Nr. 3 und 4).

Die Aufgabe der Erfindung wird durch mindestens ein Polypeptid gemäß einer der SEQ ID Nr. 1 bis SEQ ID Nr. 4, oder funktioneller Varianten davon und/oder die erfindungsgemäßen Polypeptide kodierende Nukleinsäuren oder Varianten davon sowie durch ihre Verwendung zur Diagnose, Prävention und/oder Behandlung von Erkrankungen, beispielsweise Hauterkrankungen, und/oder Diagnose und/oder Behandlung bei der Wundheilung, und/oder zur Identifizierung von pharmakologisch aktiven Substanzen gelöst.

Allgemein ist die Analyse von differentiell exprimierten Genen in Geweben mit deutlich mehr Fehlern in Form von falsch positiven Klone behaftet, als die Analyse von Zellkultursystemen. Dieses Problem kann nicht durch die Verwendung eines definierten Zellkultursystems umgangen werden, da bestehende, einfache Zellkultursysteme die Komplexität der Wundheilungsprozesse im Gewebe nicht ausreichend simulieren können.

Das Problem besteht insbesondere bei der Haut, die aus einer Vielzahl von verschiedenen Zelltypen besteht. Darüber hinaus ist der Prozeß der Wundheilung ein höchst komplizierter Vorgang, der zeitliche und räumliche Änderungen zellulärer Vorgänge, wie Proliferation und Differenzierung bei den unterschiedlichen Zelltypen umfaßt. Der Ansatz, nicht nur das komplexe Zellsystem Haut, sondern darüber hinaus den physiologischen Prozeß der Wundheilung und sogar verschiedenste Wundheilungsstadien auf der Ebene differentiell exprimierter Gene zu untersuchen ist daher für einen Fachmann keine erfolgversprechende Strategie. Der Erfolg der Rasteruntersuchung war aufgrund dieser Schwierigkeiten wesentlich von der Wahl der experimentellen Parameter abhängig. Während die verwendeten Methoden (z.B. subtraktiver Hybridisierung) Standardmethoden sind, ist die Rasteruntersuchungs- und Verifizierungsstrategie durch die durchdachte und definierte Wahl von Parametern an sich bereits erfinderisch. Beispielsweise ist der Zeitpunkt der Biopsienahme kritisch für den Erfolg der Rasteruntersuchung: Wundheilungsstörungen und Hautkrankheiten liegen häufig Störungen bei der Zellproliferation und Zellmigration zugrunde. Diese Prozesse werden einen Tag nach der Verwundung initiiert, weshalb eine Analyse der molekularen Prozesse vor diesem Zeitpunkt wenig Aufschluß über die Vorgänge liefern würde, die für eine normal verlaufende Wundheilung essentiell sind. Andererseits verändert sich im Verlauf der Wundheilung später als einen Tag nach der Verwundung die Zusammensetzung der Zelltypen in der Wunde stark. Dies kann dazu führen, daß eine differentielle Expression eines bestimmten Gens in der Wunde gemessen wird, die nicht auf veränderter Expression in den Zellen beruht, sondern nur auf der unterschiedlichen Zellzusammensetzung. Dies verdeutlicht, daß die Wahl des Tages der Biopsienahme entscheidend den Erfolg der Rasteruntersuchung beeinflußte. Trotz der definierten Parameter wurde eine Überrepräsentation von Genen beobachtet, die während der Wundheilung differentiell exprimiert werden, die aber für die Verwendung in der Wundheilung oder bei Hautkrankheiten ungeeignet sind. Diese Gene umfassen beispielsweise Gene, die für Enzyme des Primärstoffwechsels, wie Glykolyse, Citratzyklus, Glukoneogenese und Atmungskette kodieren, aber auch Gene, die für ribosomale Proteine kodieren, z.B. L41 und S20. Nur eine vergleichsweise kleine Zahl an Genen konnte als geeignet identifiziert werden. Daher war eine Identifizierung der erfindungsgemäßen GPCR-Gene als wundheilungsrelevante Gene überraschend.

Zudem gibt es enorme Variabilitäten des Wundzustands zum Zeitpunkt einer möglichen Biopsie des Patienten beim Erstkontakt mit dem Arzt. Daher wurde zur Identifikation der vorangehend beschriebenen Nukleinsäuren ein Tiermodell verwendet. Es wurden BALB/c Mäuse verwundet und zu verschiedenen Zeitpunkten Wundbiopsien entnommen. Dieses Verfahren hat den Vorteil, das sich die Randbedingungen wie genetischer Hintergrund, Art der Wunde, Zeitpunkt der Biopsie etc. exakt kontrollieren lassen und so erst eine reproduzierbare Analyse der Genexpression erlauben. Selbst unter den definierten Maus-Bedingungen ergeben sich weitere methodische Probleme wie Redundanz der analysierten Klone und Unterrepräsentation von schwach exprimierten Genen, die die Identifikation von relevanten Genen erschweren.

Allgemein ist die Analyse von differentiell exprimierten Genen in Geweben mit deutlich mehr Fehlern in Form von falsch positiven Klone behaftet, als bei der Analyse von Zellkultursystemen. Da beispielsweise bei der Wundheilung eine Vielzahl von verschiedenen Zelltypen beteiligt sind, deren Zusammensetzung und deren Genexpressionsmuster sich während des gesamten Wundheilungsprozesses ständig ändert, ergibt sich hier bei der Analyse von differentiell exprimierten Genen eine besonders niedrige Trefferquote. Diese kann nicht durch die Verwendung eines definierten Zellkultursystems umgangen werden, da ein solches auf Grund der Komplexität der Wundheilung nicht zur Verfügung steht. Zudem gibt es enorme Variabilitäten des Wundzustands zum Zeitpunkt einer möglichen Biopsie des Patienten beim Erstkontakt mit dem Arzt.

Bei der vorliegenden Analyse der Genexpression konnte SW1368 durch subtraktive Hybridisierung als wundreguliert identifiziert werden: in einer cDNA-Population, die durch Subtraktion von 1 Tages Wunden gegen intakte Haut gewonnen wurde, konnte SW1368 angereichert werden (Beispiel 1).

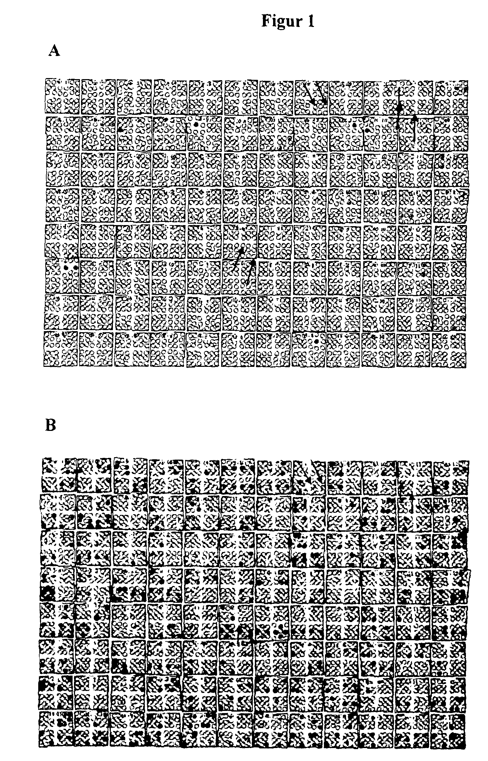

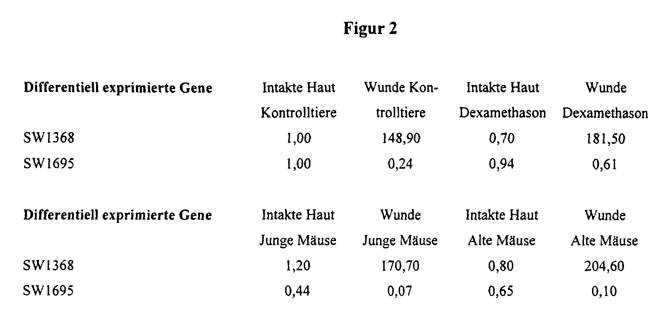

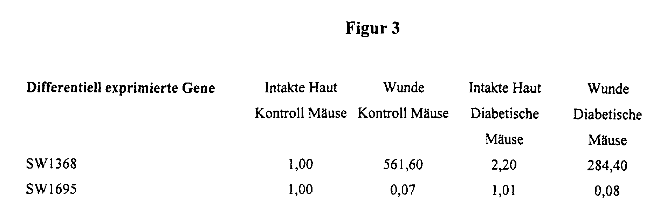

Nach der primären Identifikation der Gene ist es notwendig, die wundheilungsspezifische Expression durch eine weitere Methode zu bestätigen. Dies erfolgte mit Hilfe von sogenannten "Reverse Northern Blots" oder "TaqMan Assays". Mit diesen Methoden wurde die Menge an mRNA in Gewebeextrakten aus verschiedenen Wundheilungszuständen von 10 Wochen alten Mäusen und/oder von alten und jungen Mäusen und/oder von Mäusen mit Diabetes bestimmt. So konnte die wundspezifische Expression von SW1368 in einer subtraktiven cDNA Bibliothek mit Hilfe eines "Reverse Northern Blots" verifiziert werden (Beispiel 1, Figur 1). Zudem konnte mittels des "TaqMan Assays" die stark erhöhte Expression von SW1368 in normal heilenden Wunden von Mäusen als auch in Wunden von alten und jungen Mäusen im Vergleich zu intakter Haut quantifiziert werden (Beispiel 2; Figur 2). Zudem wurde sowohl für SW1368 als auch für SW1695, das über seine hohe Sequenzhomologie zu SW1368 mittels PCR unter Verwendung degenerierter Primern gefunden wurde, eine veränderte Expression bei anderen Wundheilungsprozessen in Maus nachgewiesen (Figur 2 und Figur 3). Während SW1368 in Wunden von Mäusen mit Diabetes um 50% schwächer exprimiert wird als in Wunden von Kontrollmäusen, konnte eine 3-fach stärkere Expression von SW1695 in schlecht heilenden Wunden von mit Dexamethason-behandelten Tieren im Vergleich zu Wunden von Kontrolltieren beobachtet werden. Dies verdeutlicht, daß die regulierte Expression der erfindungsgemäßen Genfamilie nicht nur während der normalen Wundheilung eine Rolle spielt, sondern auch für die Verhinderung krankhafter Wundheilungsverläufe notwendig ist. Die Bedeutung der erfindungsgemäßen Genfamilie konnte zudem durch "TaqMan Analyse" von humanen 1-Tages und 5-Tages Wunden untermauert werden, bei denen eine leichte, jedoch signifikante Reduktion der Expression von humanem SW1695 sowie eine Hochregulation der humanen SW1368 Expression während der Bestimmung der Wundheilungskinetik nachgewiesen werden konnte. Dagegen konnte keines der beiden Gene im Wundrand venöser Ulzera detektiert werden. Dies zeigt, daß die erfindungsgemäßen GPCRs nicht nur während der normal verlaufenden Wundheilung differentiell reguliert sind, sondern daß darüber hinaus eine Fehlregulation dieser Gene zu schweren Wundheilungsstörungen führen kann. Außerdem konnte nachgewiesen werden, daß humanes SW1695 in psoriatischer Haut fehlreguliert ist: in nicht-läsionaler Haut von Psoriasispatienten wurde eine deutlich höhere Expression im Vergleich zu läsionaler Haut gemessen. Dies zeigt, daß die Fehlregulation der erfindungsgemäßen GPCRs, insbesondere die SW1695 Expression, zur Diagnose von Hauterkrankungen, insbesondere Psoriasis, herangezogen werden kann und daß eine Fehlregulation der erfindungsgemäßen Gene Hauterkrankungen, insbesondere Psoriasis, verursachen kann.

Zur Überprüfung bzw. Generierung von Vollängen cDNA Sequenzen der vorangehend beschriebenen Nukleinsäuren wurden Vollängen-Klone mit Hilfe von Kolonie-Hybridisierung (Sambrook et al., 1989, Molecular cloning: A Laboratory Manual, Cold Spring Harbor, Cold Spring Harbor Laboratory Press, New York, Kapitel 8-10) und/oder PCR basierten Methoden ("RACE", Frohman et al., 1988, Proc. Natl. Acad. Sci. USA 85: 8998-9002, Chenchik et al., 1996, in A Laboratory Guide to RNA: Isolation, Analysis, and Synthesis, Ed. Krieg, Wiley-Liss, Seiten 272-321; "LDPCR", Barnes, 1994, Proc. Natl. Acad. Sci. USA 91: 2216-20; "IPCR", Hartl und Ochman, 1994, Methods Mol. Biol., 31: 187-196) sowohl für die Maus-Gene als auch für die menschlichen Gene generiert und die Sequenz dieser Klone bestimmt.

Der Begriff "funktionelle Varianten" eines Polypeptids im Sinne der vorliegenden Erfindung umfaßt erfindungsgemäße GPCR-Polypeptide, die beispielsweise wie die erfindungsgemäßen Polypeptide während einer Erkrankung, insbesondere Hauterkrankungen, oder bei regenerativen Prozessen der Haut, insbesondere jedoch bei der Wundheilung und krankhaften Wundheilungsstörungen und/oder Psoriasis, reguliert werden. Zu funktionellen Varianten zählen beispielsweise auch Polypeptide, die von einer Nukleinsäure kodiert werden, die aus nichthautspezifischen Gewebe, z.B. Embryonalgewebe, isoliert werden, jedoch nach Expression in einer an der Wundheilung oder Hauterkrankung beteiligten Zelle die bezeichneten Funktionen besitzen.

Funktionelle Varianten im Sinne der vorliegenden Erfindung sind auch Polypeptide, die eine Sequenzhomologie, insbesondere eine Sequenzidentität, von ca. 70%, vorzugsweise ca. 80%, insbesondere ca. 90%, vor allem ca. 95% zu dem Polypeptid mit der Aminosäuresequenz gemäß einer der SEQ ID Nr. 1 bis SEQ ID Nr. 4 aufweisen. Beispiele solcher funktioneller Varianten, sind demnach die zu einem erfindungsgemäßen Polypeptid homologen Polypeptide, die aus anderen Organismen als dem Menschen bzw. der Maus, vorzugsweise aus nichtmenschlichen Säugetieren wie z. B. Affen, Schweinen und Ratten stammen. Andere Beispiele von funktionellen Varianten sind Polypeptide, die durch unterschiedliche Allele des Gens, in verschiedenen Individuen oder in verschiedenen Organen eines Organismus kodiert werden.

Um festzustellen, ob es sich bei einem Polypeptid um eine funktionelle Variante eines erfindungsgemäßen Polypeptids handelt, kann mittels funktioneller Tests die Aktivität des zu prüfenden Polypeptids mit der Aktivität eines erfindungsgemäßen Polypeptids verglichen werden. Unter der Vorraussetzung, daß das zu prüfende Polypeptid die Anforderungen an eine funktionelle Variante auf der Ebene der Sequenzidentität erfüllt, handelt es sich bei dem zu prüfenden Polypeptid dann um eine funktionelle Variante, wenn dessen Aktivität im funktionellen Test mit der des erfindungsgemäßen Polypeptids ähnlich oder identisch ausfällt.

Diese funktionellen Tests umfassen beispielsweise die Applikation eines Expressionsvektors, der eine das zu prüfende Polypeptid kodierende Nukleinsäure enthält, oder die Applikation des zu prüfende Polypeptids selbst oder eines gegen das zu prüfende Polypeptid gerichteten Antikörpers oder eines Antisense-Oligonukleotids in Wunden. Nach Inkubation, beispielsweise eines Expressionsvektors wird der Fortgang der Wundheilung bei Gabe der unterschiedlichen Expressionsvektoren enthaltend entweder eine das zu prüfende Polypeptid kodierende Nukleinsäure oder eine erfindungsgemäßes Polypeptid kodierende Nukleinsäure oder ohne Insert verglichen. Diese funktionellen Tests können beispielsweise auch auf die Aktivität des zu prüfenden Polypeptids bei Störungen der Wundheilung, zum Beispiel bei schlecht heilenden, Dexamethason behandelten Wunden von Tieren, angewandt werden. So führte beispielsweise die Applikation von PDGF-A und PDGF-B Polypeptiden auf schlecht heilende Wunden von Kaninchen zu vergleichbarer Verbesserung der Wundheilung (J. Surg. Res., 2000, 93:230-236). Vergleichbare Tests können für Hauterkrankungen, beispielsweise Psoriasis durchgeführt werden. Dabei wird ein Expressionsvektor, der eine das zu prüfende Polypeptid kodierende Nukleinsäure enthält, oder das zu prüfende Polypeptid selbst oder ein gegen das zu prüfende Polypeptid gerichteter Antikörper oder ein Antisense-Oligonukleotid beispielsweise auf eine betroffene humane Hautpartie, die auf SCID-Mäuse transplantiert wurde, aufgetragen und der Verlauf der Hauterkrankung, beispielsweise die Heilung, ermittelt. Dies kann beispielsweise im Falle der Psoriasis durch Ermittlung des "PASI-scores" erfolgen.

Varianten des Polypeptids können auch Teile des erfindungsgemäßen Polypeptids mit mindestens 6 Aminosäuren Länge, vorzugsweise mit mindestens 8 Aminosäuren Länge, insbesondere mit mindestens 12 Aminosäuren Länge sein. Umfaßt sind auch Deletionen des erfindungsgemäßen Polypeptids im Bereich von ca. 1-30, vorzugsweise von ca. 1-15, insbesondere von ca. 1-5, Aminosäuren. Beispielsweise kann die erste Aminosäure Methionin fehlen, ohne daß die Funktion des Polypeptids wesentlich verändert wird. Weiterhin können posttranslationelle Modifikationen vorhanden sein oder fehlen, beispielsweise kovalent gebundene Lipide oder Phosphorylgruppen.

Unter "Sequenzidentität" versteht man die Übereinstimmung (% Identity) zwischen zwei Sequenzen, die im Falle von Polypeptidsequenzen beispielsweise mittels BlastP 2.0.1 und im Falle von Polynukleotidsequenzen beispielsweise mit BLASTN 2.0.14 bestimmt wird, wobei der Filter off gesetzt ist und BLOSUM 62 ist (Altschul et al., 1997, Nucleic Acids Res., 25:3389-3402). Unter "Sequenzhomologie" versteht man die Ähnlichkeit (% Positives) zwischen zwei Polypeptidsequenzen, die mittels BlastP 2.0.1 wird, wobei der Filter=off gesetzt ist und BLOSUM 62 ist (Altschul et al., 1997, Nucleic Acids Res., 25:3389-3402).

Der Begriff "kodierende Nukleinsäure" bezieht sich auf eine DNA-Sequenz, die für ein isolierbares bioaktives erfindungsgemäßes Polypeptid oder einen Precursor kodiert. Das Polypeptid kann durch eine Sequenz in voller Länge oder jeden Teil der kodierenden Sequenz kodiert werden, solange die spezifische, beispielsweise Rezeptor-Aktivität erhalten bleibt.

Es ist bekannt, daß kleine Veränderungen in der Sequenz der vorangehend beschriebenen Nukleinsäuren vorhanden sein können, zum Beispiel durch die Degenerierung des genetischen Codes, oder daß nicht translatierte Sequenzen am 5' und/oder 3'-Ende der Nukleinsäure angehängt sein können, ohne daß dessen Aktivität wesentlich verändert wird. Diese Erfindung umfaßt deshalb auch sogenannte "Varianten" der vorangehend beschriebenen Nukleinsäuren.

Unter Varianten der Nukleinsäuren sind alle DNA-Sequenzen zu verstehen, die komplementär zu einer DNA-Sequenz sind, die unter stringenten Bedingungen mit der Referenzsequenz hybridisieren und eine ähnliche Aktivität aufweisen wie das von der Referenzsequenz kodierte Polypeptid.

Unter "stringenten Hybridisierungsbedingungen" sind solche Bedingungen zu verstehen, bei denen eine Hybridisierung bei 60°C in 2,5 x SSC-Puffer, gefolgt von mehreren Waschschritten bei 37°C in einer geringeren Pufferkonzentration erfolgt und stabil bleibt.

Varianten der Nukleinsäure können auch Teile der erfindungsgemäßen Nukleinsäure mit mindestens 8 Nukleotiden Länge, vorzugsweise mit mindestens 18 Nukleotiden Länge, insbesondere mit mindestens 24 Nukleotiden Länge sein.

Unter dem Begriff "pharmakologisch aktive Substanz" im Sinne der vorliegenden Erfindung sind alle diejenigen Moleküle, Verbindungen und/oder Zusammensetzungen und Stoffgemische zu verstehen, die mit den erfindungsgemäßen Nukleinsäuren, Polypeptiden, Antikörpern oder Antikörperfragmenten, gegebenenfalls zusammen mit geeigneten Zusatz- und/oder Hilfsstoffen, unter geeigneten Bedingungen in Wechselwirkung treten können. Mögliche pharmakologisch aktive Substanzen sind einfache chemische (organische oder anorganische) Moleküle oder Verbindungen, können aber auch Nukleinsäuren, Peptide, Proteine oder Komplexe davon umfassen. Beispiele pharmakologisch aktiver Substanzen sind organische Moleküle, die Substanzbibliotheken entstammen, die auf ihre pharmakologische Aktivität hin untersucht wurden. Die pharmakologisch aktiven Substanzen können aufgrund ihrer Wechselwirkung die Funktion(en) der Nukleinsäuren, Polypeptide oder Antikörper in vivo oder in vitro beeinflussen oder auch nur an die vorangehend beschriebenen Nukleinsäuren, Polypeptide, Antikörpern oder Antikörperfragmente binden oder mit ihnen andere Wechselwirkungen kovalenter oder nicht-kovalenter Weise eingehen.

Die Erfindung betrifft erfindungsgemäße GPCR-Polypeptide oder Varianten davon gemäß einer der SEQ ID Nr. 1 bis SEQ ID Nr. 4 und/oder diese kodierende Nukleinsäuren oder Varianten davon.

Die vorangehend beschriebenen Polypeptide können dadurch gekennzeichnet sein, daß sie synthetisch hergestellt werden. So kann das gesamte Polypeptid oder Teile davon zum Beispiel mit Hilfe der klassischen Synthese (Merrifield-Technik) synthetisiert werden. Teile der vorangehend beschriebenen Polypeptide eignen sich insbesondere zur Gewinnung von Antiseren, mit deren Hilfe geeignete Genexpressionsbanken durchsucht werden können, um so zu weiteren Varianten, vorzugsweise funktionellen Varianten, der vorangehend beschriebenen Polypeptide zu gelangen.

Bevorzugterweise handelt es sich bei den erfindungsgemäßen Nukleinsäuren um DNA oder RNA, vorzugsweise eine DNA, insbesondere eine doppelsträngige DNA. Weiterhin kann die Sequenz der Nukleinsäuren dadurch gekennzeichnet sein, daß sie mindestens ein Intron und/oder eine polyA-Sequenz aufweist. Die erfindungsgemäßen Nukleinsäuren können auch in Form ihrer antisense-Sequenz verwendet werden.

Für die Expression des betreffenden Gens ist im allgemeinen eine doppelsträngige DNA bevorzugt, wobei der für das Polypeptid kodierende DNA-Bereich besonders bevorzugt ist. Dieser Bereich beginnt bei Eukaryonten mit dem ersten in einer Kozak Sequenz (Kozak, 1987, Nucleic. Acids Res. 15:8125-48) liegenden Start-Codon (ATG) bis zum nächsten Stop-Codon (TAG, TGA bzw. TAA), das im gleichen Leseraster zum ATG liegt. Bei Prokaryonten beginnt dieser Bereich mit dem ersten AUG (oder GUG) nach einer Shine-Dalgarno-Sequenz und endet mit dem nächsten Stop-Codon (TAA, TAG bzw. TGA), das im gleichen Leseraster zum ATG liegt.

Weiterhin können die erfindungsgemäßen Nukleinsäuresequenzen für die Konstruktion von anti-sense Oligonukleotiden (Zheng und Kemeny, 1995, Clin. Exp. Immunol. 100:380-2; Nellen und Lichtenstein, 1993, Trends Biochem. Sci. 18:419-23; Stein, 1992, Leukemia 6:967-74) und/oder Ribozymen (Amarzguioui, et al. 1998, Cell. Mol. Life Sci. 54:1175-202; Vaish, et al., 1998, Nucleic Acids Res. 26:5237-42; Persidis, 1997, Nat. Biotechnol. 15:921-2; Couture und Stinchcomb, 1996, Trends Genet. 12:510-5) herangezogen werden. Mit anti-sense Oligonukleotiden kann man die Stabilität von Nukleinsäuren verringert werden und/oder die Translation von Nukleinsäuren inhibiert werden. So kann durch die Verwendung der Nukleinsäuresequenzen beispielsweise die Expression der entsprechenden Gene in Zellen sowohl *in vivo* als auch *in vitro* verringert werden. Oligonukleotide können sich daher als Therapeutikum eignen. Diese Strategie eignet sich beispielsweise auch für Haut, epidermale und dermale Zellen, insbesondere, wenn die antisense Oligonukleotide mit Liposomen komplexiert werden (Smyth et al., 1997, J. Invest. Dermatol. 108:523-6; White et al., 1999, J. Invest. Dermatol. 112:699-705; White et al., 1999, J. Invest. Dermatol. 112:887-92). Für die Verwendung als Sonde oder als "antisense" Oligonukleotid ist eine einzelsträngige DNA oder RNA bevorzugt.

Weiterhin kann zur Durchführung der Erfindung eine Nukleinsäure verwendet werden, die synthetisch hergestellt worden ist. So kann die erfindungsgemäße Nukleinsäure beispielsweise chemisch anhand der in SEQ ID Nr. 5 bis SEQ ID Nr. 8 beschriebenen DNA Sequenzen und/oder anhand der in SEQ ID Nr. 1 bis SEQ ID Nr. 4 beschriebenen Proteinsequenzen unter Heranziehen des genetischen Codes z. B. nach der Phosphotriester-Methode synthetisiert werden (siehe z. B. Uhlmann, E. & Peyman, A. (1990) Chemical Revievs, 90, 543-584, No. 4).

Oligonukleotide werden in der Regel schnell durch Endo- oder Exonukleasen, insbesondere durch in der Zelle vorkommende DNasen und RNasen, abgebaut. Deshalb ist es vorteilhaft, die Nukleinsäure zu modifizieren, um sie gegen den Abbau zu stabilisieren, so daß über einen langen Zeitraum eine hohe Konzentration der Nukleinsäure in der Zelle beibehalten wird (Beigelman et al., 1995, Nucleic Acids Res. 23:3989-94; Dudycz, 1995, WO 95/11910; Macadam et al., 1998, WO 98/37240; Reese et al., 1997, WO 97/29116). Typischerweise kann eine solche Stabilisierung durch die Einführung von einer oder mehrerer Internukleotid-Phosphorgruppen oder durch die Einführung einer oder mehrerer Nicht-Phosphor-Internukleotide, erhalten werden.

Geeignete modifizierte Internukleotide sind in Uhlmann und Peymann (1990 Chem. Rev. 90, 544) zusammengefaßt (siehe auch Beigelman et al., 1995 Nucleic Acids Res. 23: 3989-94; Dudycz, 1995, WO 95/11910; Macadam et al., 1998, WO 98/37240; Reese et al., 1997, WO 97/29116). Modifizierte Internukleotid-Phosphatreste und/oder Nicht-Phosphorbrücken in einer Nukleinsäure, die bei einer der erfindungsgemäßen Verwendungen eingesetzt werden können, enthalten zum Beispiel Methylphosphonat, Phosphorothioat, Phosphoramidat, Phosphorodithioat, Phophatester, während Nicht-Phosphor-Internukleotid-Analoge, beispielsweise Siloxanbrücken, Carbonatbrücken, Carboxymethylester, Acetamidatbrücken und/oder Thioetherbrücken enthalten. Es ist auch beabsichtigt, daß diese Modifizierung die Haltbarkeit einer pharmazeutischen Zusammensetzung, die bei einer der erfindungsgemäßen Verwendungen eingesetzt werden kann, verbessert.

In einer weiteren Ausführungsform sind die erfindungsgemäßen Nukleinsäuren in einem Vektor, vorzugsweise in "Shuttle" -Vektoren, Phagemiden, Cosmiden, Expressionsvektoren oder gentherapeutisch wirksamen Vektoren enthalten. Weiterhin können die vorangehend beschriebenen Nukleinsäuren in "knock-out" Genkonstrukten oder Expressionskassetten enthalten sein. Eine Expressionskassette im Sinne der vorliegenden Erfindung umfaßt mindestens einen Promotor oder Enhancer, mindestens ein Translationsinitiationssignal, mindestens eine der vorangehend beschriebenen Nukleinsäuren, ein Translationsterminierungssignal, ein Transkriptionsterminierungssignal sowie ein Polyadenylierungssignal für die Expression in Eukaryonten.

Vorzugsweise enthält ein gentherapeutisch wirksamer Vektor wund- bzw. hautspezifische regulatorische Sequenzen, die funktionell mit der vorangehend beschriebenen Nukleinsäure verbunden sind.

Geeignete Expressionsvektoren können prokaryotische oder eukaryotische Expressionsvektoren sein. Beispiele für prokaryotische Expressionsvektoren sind für die Expression in *E. coli* z.B. die Vektoren pGEM oder pUC-Derivate und für eukaryotische Expressionsvektoren für die Expression in *Saccharomyces cerevisiae* z. B. die Vektoren p426Met25 oder p426GAL1 (Mumberg et al. (1994) Nucl. Acids Res., 22, 5767-5768), für die Expression in Insektenzellen z. B. *Baculovirus*-Vektoren wie in EP-B1-0 127 839 oder EP-B1-0 549 721 offenbart, und für die Expression in Säugerzellen z. B. die Vektoren Rc/CMV und Rc/RSV oder SV40-Vektoren, welche alle allgemein erhältlich sind.

Im allgemeinen enthalten die Expressionsvektoren auch für die jeweilige Zelle geeignete Promotoren, wie z. B. den trp-Promotor für die Expression in *E. coli* (siehe z. B. EP-B1-0 154 133), den MET25, GAL1 oder ADH2-Promotor fiir die Expression in Hefen (Russel et al. (1983), J. Biol. Chem. 258, 2674-2682; Mumberg, supra), den Baculovirus-Polyhedrin-Promotor, für die Expression in Insektenzellen (siehe z. 13. EP-B1-0 127 839). Für die Expression in Säugetierzellen sind beispielsweise Promotoren geeignet, die eine konstitutive, regulierbare, gewebsspezifische, zellzyklusspezifische oder metabolischspezifische Expression in eukaryotischen Zellen erlauben. Regulierbare Elemente gemäß der vorliegenden Erfindung sind Promotoren, Aktivatorsequenzen, Enhancer, Silencer und/oder Repressorsequenzen.

Beispiel für geeignete regulierbare Elemente, die konstitutive Expression in Eukaryonten ermöglichen, sind Promotoren, die von der RNA Polymerase III erkannt werden oder virale Promotoren, CMV-Enhancer, CMV-Promotor, SV40 Promotor oder LTR-Promotoren z. B. von MMTV (mouse mammary tumour virus; Lee et al. (1981) Nature 214, 228-232) und weitere virale Promotor- und Aktivatorsequenzen, abgeleitet aus beispielsweise HBV, HCV, HSV, HPV, EBV, HTLV oder HIV.

Beispiele für regulierbare Elemente, die regulierbare Expression in Eukaryonten ermöglichen, sind der Tetracyclinoperator in Kombination mit einem entsprechenden Repressor (Gossen M. et al. (1994) Curr. Opin. Biotechnol. 5, 516-20).

Vorzugsweise erfolgt die Expression von wundheilungsrelevanten Genen unter der Kontrolle von gewebespezifischen Promotoren, beispielsweise unter Kontrolle von hautspezifischen Promotoren wie beispielsweise dem humanen K10 Promotor (Bailleul et al., 1990. Cell 62: 697-708), dem humanen K14 Promotor (Vassar et al., 1989, Proc. Natl. Acad. Sci. USA 86: 1563-67) oder dem bovinen Cytokeratin IV Promotor (Fuchs et al., 1988; The biology of wool and hair (Hrsg.: G.E. Rogers, et al.; S. 287-309; Chapman und Hall, London/New York).

Weitere Beispiele für regulierbare Elemente, die gewebespezifische Expression in Eukaryonten ermöglichen, sind Promotoren oder Aktivatorsequenzen aus Promotoren oder Enhancern von solchen Genen, die für Proteine kodieren, die nur in bestimmten Zelltypen exprimiert werden.

Beispiele für regulierbare Elemente, die zellzyklusspezifische Expression in Eukaryonten ermöglichen, sind Promotoren folgender Gene: cdc25A, cdc25B, cdc25C, Cyclin A, Cyclin E, cdc2, E2F-1 bis E2F-5, B-myb oder DHFR (Zwicker J. und Müller R. (1997) Trends Genet. 13, 3-6). Die Verwendung von zellzyklusregulierten Promotoren ist besonders bevorzugt in Fällen, in denen die Expression der erfindungsgemäßen Polypeptide oder Nukleinsäuren auf proliferierende Zellen beschränkt werden soll.

Beispiele für regulierbare Elemente, die metabolischspezifische Expression in Eukaryonten ermöglichen, sind Promotoren, die durch Hypoxie, durch Glukosemangel, durch Phosphatkonzentration oder durch Hitzeschock reguliert werden.

Ein Beispiel fiir ein regulierbares Element, das die Keratinozyten-spezifische Expression in der Haut erlaubt, ist das FiRE-Element (Jaakkola et al., 2000, Gen. Ther., 7: 1640-1647). Das FiRE Element ist AP-1-getriebenes, durch FGF-induzierbares sogenanntes Response Element des Syndecan-1 Gens (Jaakkola et al., 1998, FASEB J., 12: 959-9).

Um die Einführung von den vorangehend beschriebenen Nukleinsäuren und damit die Expression des Polypeptids in einer eu- oder prokaryotischen Zelle durch Transfektion, Transformation oder Infektion zu ermöglichen, kann die Nukleinsäure als Plasmid, als Teil eines viralen oder nicht-viralen Vektors vorliegen. Als virale Vektoren eignen sich hierbei besonders: Baculoviren, Vakziniaviren, Adenoviren, adenoassoziierte Viren und Herpesviren. Als nicht-virale Vektoren eignen sich hierbei besonders: Virosomen, Liposomen, kationische Lipide, oder poly-Lysin konjugierte DNA.

Beispiele von gentherapeutisch wirksamen Vektoren sind Virusvektoren, beispielsweise Adenovirusvektoren, retroviralen Vektoren oder Vektoren die auf Replikons von RNA Viren beruhen (Lindemann et al., 1997, Mol. Med. 3: 466-76; Springer et al., 1998, Mol. Cell. 2: 549-58; Khromykh, 2000, Curr. Opin. Mol. Ther.;2:555-69). Eukaryotische Expressionsvektoren eignen sich in isolierter Form für die gentherapeutische Anwendung, da nackte DNA bei topischer Applikation beispielsweise in Hautzellen eindringen kann (Hengge et al., 1996, J. Clin. Invest. 97: 2911-6; Yu et al., 1999, J. Invest. Dermatol. 112: 370-5).

Gentherapeutisch wirksame Vektoren lassen sich auch dadurch erhalten, daß man die vorangehend beschriebenen Nukleinsäure mit Liposomen komplexiert, da damit eine sehr hohe Transfektionseffizienz, beispielsweise von Hautzellen, erreicht werden kann (Alexander und Akhurst, 1995, Hum. Mol. Genet. 4: 2279-85). Bei der Lipofektion werden kleine unilamellare Vesikel aus kationischen Lipiden durch Ultraschallbehandlung der Liposomensuspension herstellt. Die DNA wird ionisch auf der Oberfläche der Liposomen gebunden, und zwar in einem solchen Verhältnis, daß eine positive Nettoladung verbleibt und die Plasmid-DNA zu 100% von den Liposomen komplexiert wird. Neben den von Felgner et al. (1987, supra) eingesetzten Lipidmischungen DOTMA (1,2-Dioleyloxypropyl-3-trimethylammoniumbromid) und DPOE (Dioleoylphosphatidylethanolamin) wurden inzwischen zahlreiche neue Lipidformulierungen synthetisiert und auf ihre Effizienz der Transfektion verschiedener Zellinien getestet (Behr et al. (1989), Proc. Natl. Acad. Sci. USA 86, 6982-6986; Gao und Huang (1991), Biochim. Biophys. Acta 1189, 195-203; Felgner et al. (1994) J. Biol. Chem. 269, 2550-2561). Beispiele der neuen Lipidformulierungen sind DOTAP N-[1-(2,3-Dioleoyloxy)propyl]-N,N,N-trimethylammoniumethylsulfat oder DOGS (TRANSFECTAM; Dioctadecylamidoglycylspermin). Als sehr gut geeignet für die Transfektion von Keratinozyten in vitro und in vivo haben sich auch die kationischen Lipide Cytofectin GS 2888 erwiesen (US 5,777,153; Lewis et al., 1996, Proc. Natl. Acad. Sci. USA, 93: 3176-3181). Hilfsstoffe, die den Transfer von Nukleinsäuren in die Zelle erhöhen, können beispielsweise Proteine oder Peptide, die an DNA gebunden sind oder synthetische Peptid-DNA-Moleküle, die den Transport der Nukleinsäure in den Kern der Zelle ermöglichen, sein (Schwartz et al. (1999) Gene Therapy 6, 282; Brandén et al. (1999) Nature Biotech. 17, 784). Hilfsstoffe umfassen auch Moleküle, die die Freisetzung von Nukleinsäuren in das Cytoplasma der Zelle ermöglichen (Planck et al. (1994) J. Biol. Chem. 269, 12918; Kichler et al. (1997) Bioconj. Chem. 8, 213) oder beispielsweise Liposomen (Uhlmann und Peymann (1990) supra). Eine andere besonders geeignete Form von gentherapeutischen Vektoren läßt sich dadurch erhalten, daß man die vorangehend beschriebene Nukleinsäure auf Goldpartikeln aufbringt und diese mit Hilfe der sogenannten "Gene Gun" in Gewebe, beispielsweise in die Haut, oder Zellen schießt (Wang et al., 1999, J. Invest. Dermatol., 112:775-81, Tuting et al., 1998, J. Invest. Dermatol. 111:183-8).

Eine weitere Form eines gentherapeutisch wirksamen Vektors läßt sich durch das Einbringen von "nackten" Expressionsvektoren in eine biokompatible Matrix, beispielsweise eine Kollagenmatrix, herstellen. Diese Matrix kann beispielsweise in Wunden eingebracht werden, um die einwandernden Zellen mit dem Expressionsvektor zu transfizieren und die erfindungsgemäßen Polypeptide in den Zellen zu exprimieren (Goldstein und Banadio, US 5,962,427).

Für die gentherapeutische Anwendung der vorangehend beschriebenen Nukleinsäure ist es auch von Vorteil, wenn der Teil der Nukleinsäure, der für das Polypeptid kodiert, ein oder mehrere nicht kodierende Sequenzen einschließlich Intronsequenzen, vorzugsweise zwischen Promotor und dem Startcodon des Polypeptids, und/oder eine polyA-Sequenz, insbesondere die natürlich vorkommende polyA-Sequenz oder eine SV40 Virus polyA-Sequenz, vor allem am 3'-Ende des Gens enthält, da hierdurch eine Stabilisierung der mRNA erreicht werden kann (Palmiter et al., 1991, Proc. Natl. Acad. Sci. USA 88:478-482; Jackson,1993, Cell 74:9-14).

Knock-out Genkonstrukte sind dem Fachmann zum Beispiel aus den US-Patenten 5,625,122; US 5,698,765; US 5,583,278 und US 5,750,825 bekannt.

Ein weiterer Gegenstand der vorliegenden Erfindung ist eine Zelle, beispielsweise eine Hautzelle, die mit einem der vorangehend beschriebenen Vektoren, Expressionskassette, und/oder knock-out Genkonstrukt transformiert ist. Zellen können sowohl prokaryotische als auch eukaryotische Zellen, heterologe oder autologe Zellen sein. Beispiele für prokaryotische Zellen sind *E. coli* und für eukaryotische Zellen sind Hautzellen, Keratinozyten, Fibroblasten oder Endothelzellen, Hefezellen, beispielsweise *Saccharomyces cerevisiae* oder Insektenzellen.

Ein besonders bevorzugte transformierte Zelle ist eine transgene embryonale nichtmenschliche Stammzelle, die mindestens eine erfindungsgemäß verwendbare Nukleinsäure, mindestens einen Vektor, mindestens ein knock-out Genkonstrukt, und/oder mindestens eine Expressionskassette, wie vorangehend beschrieben, enthält. Verfahren zur Transformation von Zellen und/oder Stammzellen sind dem Fachmann gut bekannt und umfassen zum Beispiel Elektroporation oder Mikroinjektion.

Ein weiterer Gegenstand der Erfindung ist ein transgenes nichtmenschliches Säugetier, dessen Genom mindestens eine erfindungsgemäß verwendbare Nukleinsäure, mindestens einen Vektor, mindestens ein knock-out Genkonstrukt, und/oder mindestens eine Expressionskassette, wie vorangehend beschrieben, enthält. Transgene Tiere zeigen im allgemeinen eine gewebespezifisch erhöhte Expression der Nukleinsäuren und/oder Polypeptide und lassen sich zur Analyse beispielsweise von Wundheilungsstörungen verwenden. So weist beispielsweise eine Activin A transgene Maus eine verbesserte Wundheilung auf (Munz et al., 1999, EMBO J. 18:5205-15) während eine transgene Maus mit dominant negativem KGF Rezeptor eine verzögerte Wundheilung aufweist (Werner et al., 1994, Science 266:819-22).

Verfahren zur Herstellung von transgenen Tieren, insbesondere von transgenen Mäusen, sind dem Fachmann ebenfalls aus der DE 196 25 049 und den US 4,736,866; US 5,625,122; US 5,698,765; US 5,583,278 und US 5,750,825 bekannt und umfassen transgene Tiere, die beispielsweise über direkte Injektion von erfindungsgemäßen Expressionsvektoren in Embryonen oder Spermatozyten oder über die Transfektion von Expressionsvektoren in embryonale Stammzellen erzeugt werden können (Polites und Pinkert: DNA Microinjection and Transgenic Animal Produktion, Seite 15 bis 68 in Pinkert, 1994: Transgenic animal technology: A Laboratory Handbook, Academic Press, London, UK; Houdebine, 1997, Harwood Academic Publishers, Amsterdam, The Netherlands; Doetschman: Gene Transfer in Embryonic Stem Cells, Seite 115 bis 146 in Pinkert, 1994, supra; Wood: Retrovirus-Mediated Gene Transfer, Seite 147 bis 176 in Pinkert, 1994, supra; Monastersky: Gene Transfer Technology: Alternative Techniques and Applications, Seite 177 bis 220 in Pinkert, 1994, supra).

Werden die vorangehend beschriebenen Nukleinsäuren in sogenannte "Targeting" Vektoren oder "knock-out" Genkonstrukte integriert (Pinkert, 1994, supra) können nach Transfektion von embryonalen Stammzellen und homologer Rekombination beispielsweise knock-out Mäuse generiert werden, die im allgemeinen als heterozygote Mäuse verringerte Expression der Nukleinsäure zeigen, während homozygote Mäuse keine Expression der Nukleinsäure mehr aufweisen. Auch die so erzeugten Tiere lassen sich zur Analyse beispielsweise von Wundheilungsstörungen verwenden. So weisen beispielsweise die eNOS- (Lee et al., 1999, Am. J. Physiol. 277:H1600-H1608), Nf-1 (Atit et al., 1999, J. Invest. Dermatol. 112:835-42) und Osteopontin (Liaw et al., 1998, J. Clin. Invest. 101:967-71) knock-out Mäuse eine verschlechterte Wundheilung auf. Auch hier ist eine gewebespezifische Reduktion der Expression wundheilungsrelevanter Gene, beispielsweise in hautspezifischen Zellen unter Verwendung des Cre-loxP Systems (stat3 knock-out, Sano et al., EMBO J. 1999 18:4657-68), besonders zu bevorzugen. So erzeugte transgene und knock-out Zellen oder Tiere lassen sich auch zum Screening und zur Identifizierung von pharmakologisch aktiven Substanzen bzw. gentherapeutisch aktiven Vektoren verwenden.

Ein weiterer Gegenstand der Erfindung betrifft ein Verfahren zur Herstellung eines erfindungsgemäßen Polypeptids, das beispielsweise zur Diagnose und/oder Behandlung von Erkrankungen, beispielsweise Hauterkrankungen, und/oder Diagnose und/oder Behandlung bei der Wundheilung und/oder dessen krankhaften Störungen, und/oder zur Identifizierung von pharmakologisch aktiven Substanzen verwendet werden kann, wobei in einer geeigneten Zellemindestens eine der erfindungsgemäßen Nukleinsäuren exprimiert und das Polypeptid gegebenenfalls isoliert wird.

Das erfindungsgemäße Polypeptid wird beispielsweise durch Expression der vorangehend beschriebenen Nukleinsäuren in einem geeigneten Expressionssystem, wie oben bereits erwähnt, nach dem Fachmann allgemein bekannten Methoden hergestellt. Als Zellen eignen sich beispielsweise die *E. coli* Stämme DHS, HB101 oder BL21, der Hefestamm *Saccharomyces cerevisiae*, die Insektenzellinie Lepidopteran, z. B. von *Spodoptera frugiperda*, oder die tierischen Zellen COS, Vero, 293, HaCaT, und HeLa, die alle allgemein erhältlich sind.

Ein weiterer Gegenstand der Erfindung ist ein Verfahren zur Herstellung eines erfindungsgemäßen Fusionsproteins, das beispielsweise zur Diagnose und/oder Behandlung von Erkrankungen, beispielsweise Hauterkrankungen, und/oder Diagnose und/oder Behandlung bei der Wundheilung und/oder dessen krankhaften Störungen, und/oder zur Identifizierung von pharmakologisch aktiven Substanzen verwendet werden kann, in einer geeigneten Zelle, bei dem mindestens eine der vorangehend beschriebenen Nukleinsäuren verwendet wird.

Hergestellt werden hierbei Fusionsproteine, die die oben beschriebenen Polypeptide enthalten, wobei die Fusionsproteine selbst bereits die Funktion eines Polypeptids der Erfindung aufweisen oder erst nach Abspaltung des Fusionsanteils die spezifische Funktion aufweisen. Vor allem zählen hierzu Fusionsproteine mit einem Anteil von ca. 1-300, vorzugsweise ca. 1-200, insbesondere ca. 1-100, vor allem ca. 1-50 fremden Aminosäuren. Beispiele solcher Peptidsequenzen sind prokaryotische Peptidsequenzen, die z. B. aus der Galactosidase von *E. coli* abgeleitet sein können. Weiterhin können auch virale Peptidsequenzen, wie zum Beispiel vom Bakteriophagen M13 verwendet werden, um so Fusionsproteine für das dem Fachmann bekannte "phage display"-Verfahren zu erzeugen. Die oben erwähnten Fusionsproteine sind ebenfalls Gegenstand der vorliegenden Erfindung.

Weitere bevorzugte Beispiele für Peptidsequenzen für Fusionsproteine sind Peptide, die die Detektion der Fusionsproteine erleichtern, hierzu zählen beispielsweise "Green-fluorescent-protein" oder Varianten davon.

Zur Aufreinigung der vorangehend beschriebenen Proteine kann/können ein weiteres/weitere Polypeptid ("tag/tags") angefügt sein. Erfindungsgemäße Protein-tags erlauben beispielsweise die hochaffine Absorption an eine Matrix, stringentes Waschen mit geeigneten Puffern, ohne den Komplex in nennenswertem Maße zu eluieren und anschließend gezielte Elution des absorbierten Komplexes. Beispiele der dem Fachmann bekannten Protein-tags sind ein (His)₆-tag, ein Myc-tag, ein FLAG-tag, ein Hämagglutinin-tag, Glutathion-Transferase (GST)-tag, Intein mit einem Affinitäts-Chitin-binding-tag oder Maltose-binding protein (MBP)-tag. Diese Protein-tags können sich N-, C-terminal und/oder intern befinden.

Ein weiterer Gegenstand der Erfindung betrifft ein Verfahren zur Herstellung eines Antikörpers oder Antikörperfragments, vorzugsweise eines polyklonalen oder monoklonalen Antikörpers, der beispielsweise zur Diagnose und/oder Prävention und/oder Behandlung von Erkrankungen, beispielsweise Hauterkrankungen, und/oder Diagnose und/oder Behandlung bei der Wundheilung und/oder dessen krankhaften Störungen, oder zur Identifizierung von pharmakologisch aktiven Substanzen verwendet werden kann.

Das Verfahren erfolgt nach dem Fachmann allgemein bekannten Methoden durch Immunisieren eines Säugetiers, beispielsweise eines Kaninchens, mit einer erfindungsgemäßen Nukleinsäure, oder mit einem erfindungsgemäßen Polypeptid oder funktionellen Varianten davon, vorzugsweise mit Teilen davon, mit mindestens 6 Aminosäuren Länge, vorzugsweise mit mindestens 8 Aminosäuren Länge, insbesondere mit mindestens 12 Aminosäuren Länge, gegebenenfalls in Anwesenheit von z. B. Freund's Adjuvant und/oder Aluminiumhydroxidgelen (siehe z. B. Diamond, B. A. et al. (1981) The New England Journal of Medicine, 1344-1349). Die im Tier aufgrund einer immunologischen Reaktion entstandenen polyklonalen Antikörper lassen sich anschließend nach allgemein bekannten Methoden leicht aus dem Blut isolieren und z. B. über Säulenchromatographie reinigen. Monoklonale Antikörper können beispielsweise nach der bekannten Methode von Winter & Milstein (Winter, G. & Milstein, C. (1991) Nature, 349, 293-299) hergestellt werden.

Ein weiterer Gegenstand der vorliegenden Erfindung ist ein Antikörper oder Antikörperfragment, der gegen ein vorangehend beschriebenes Polypeptid gerichtet ist und mit dem vorangehend beschriebenen Polypeptiden spezifisch reagiert, wobei die oben genannten Teile des Polypeptids entweder selbst immunogen sind oder durch Kopplung an geeignete Träger, wie z.B. bovines Serumalbumin, immunogen gemacht bzw. in ihrer Immunogenität gesteigert werden können. Dieser Antikörper ist entweder polyklonal oder monoklonal, bevorzugt ist ein monoklonaler Antikörper. Unter dem Begriff Antikörper oder Antikörperfragment versteht man gemäß der vorliegenden Erfindung auch gentechnisch hergestellte und gegebenenfalls modifizierte Antikörper bzw. antigenbindende Teile davon, wie z.B. chimäre Antikörper, humanisierte Antikörper, multifunktionelle Antikörper, bi- oder oligospezifische Antikörper, einzelsträngige Antikörper, F(ab)- oder F(ab)₂-Fragmente (siehe z.B. EP-B1-0 368 684, US 4,816,567, US 4,816,397, WO 88/01649, WO 93/06213, WO 98/24884). Die erfindungsgemäßen Antikörper können beispielsweise zur Diagnose und/oder Prävention und/oder Behandlung von Erkrankungen, beispielsweise Hauterkrankungen, und/oder Diagnose und/oder Behandlung bei der Wundheilung und/oder dessen krankhaften Störungen, und/oder zur Identifizierung von pharmakologisch aktiven Substanzen verwendet werden.

Als Alternative zu den klassischen Antikörpern können beispielsweise auf Lipocalin basierende sogenannte "Anticaline" verwendet werden (Beste et al., 1999, Proc. Natl. Acad. Sci. USA, 96:1898-1903). Die natürlichen Liganden-bindenden Sites der Lipocaline, wie z.B. das Retinol-bindende Protein oder das Bilinbindende Protein können beispielsweise durch einen "kombinatorischen Protein Design" Ansatz in einer Weise verändert werden, daß sie an ausgewählte Haptene, beispielsweise an die erfindungsgemäß verwendbaren Polypeptide binden (Skerra, 2000, Biochim. Biophys. Acta 1482:337-50). Weitere bekannte "scaffolds" als Alternativen für Antikörper fiir die molekulare Erkennung sind bekannt (Skerra, J. Mol. Recognit., 2000, 13:167-187).

So kann beispielsweise die lokale Injektion von monoklonalen Antikörpern gegen TGF beta 1 im Tiermodell die Wundheilung verbessern (Ernst et al., 1996, Gut 39:172-5).

Die vorliegende Erfindung umfaßt ferner die Verwendung mindestens eines erfindungsgemäßen Polypeptids, und/oder mindestens einer erfindungsgemäßen Fusionsprotein, und/oder mindestens einer erfindungsgemäßen Nukleinsäure, und/oder mindestens einer erfindungsgemäßen Zelle, und/oder mindestens eines erfindungsgemäßen Antikörpers oder Antikörperfragments, zur Herstellung eines pharmazeutischen Präparates zur Diagnose, Prävention und/oder Behandlung von Hauterkrankungen, und/oder zur Diagnose und/oder Behandlung bei der Wundheilung und/oder deren krankhaften Störungen. Pharmazeutische Präparate im Sinne der Erfindung umfassen Arzneimittel und Diagnostika, die zur Prävention, Diagnose und Behandlung von Erkrankungen verwendet werden können.

Die vorliegende Erfindung betrifft auch ein Verfahren zur Herstellung eines Arzneimittels zur Behandlung und/oder Prävention von Erkrankungen, beispielsweise Hauterkrankungen, und/oder Behandlung bei der Wundheilung und/oder dessen krankhaften Störungen, bei dem mindestens eine erfindungsgemäße Nukleinsäure, mindestens ein erfindungsgemäßes Polypeptid, mindestens ein erfindungsgemäßer Antikörper oder Antikörperfragment und/oder eine erfindungsgemäße Zelle, zusammen mit geeigneten Zusatz- und Hilfsstoffen kombiniert wird.

Die vorliegende Erfindung betrifft weiterhin ein nach diesem Verfahren hergestelltes Arzneimittel zur Behandlung und/oder Prävention von Erkrankungen, beispielsweise Hauterkrankungen und/oder Behandlung und/oder Prävention bei der Wundheilung, und/oder dessen krankhaften Störungen, das mindestens eine erfindungsgemäße Nukleinsäure, mindestens ein erfindungsgemäßes Polypeptid oder mindestens einen erfindungsgemäßen Antikörper oder Antikörperfragment oder eine erfindungsgemäße Zelle, gegebenenfalls zusammen mit geeigneten Zusatz- und Hilfsstoffen, enthält. Die Erfindung betrifft weiterhin die Verwendung dieses Arzneimittels zur Behandlung von Erkrankungen, beispielsweise Hauterkrankungen, und/oder bei der Wundheilung und/oder dessen krankhaften Störungen.

Für die gentherapeutische Anwendung bei Hauterkrankungen und/oder der Wundheilung, beispielsweise einer gestörten Wundheilung beim Menschen ist vor allem ein Arzneimittel geeignet, das die beschriebenen Nukleinsäure in nackter Form oder in Form eines der oben beschriebenen gentherapeutisch wirksamen Vektoren oder in mit Liposomen bzw. Goldpartikeln komplexierter Form enthält. Der pharmazeutische Träger ist beispielsweise eine physiologische Pufferlösung, vorzugsweise mit einem pH von ca. 6,0-8,0, vorzugsweise von ca. 6,8-7,8. Insbesondere von ca. 7,4 und/oder einer Osmolarität von ca. 200-400 milliosmol/Liter, vorzugsweise von ca. 290-310 milliosmol/Liter. Zusätzlich kann der pharmazeutische Träger geeignete Stabilisatoren, wie z.B. Nukleaseinhibitoren, vorzugsweise Komplexbildner wie EDTA und/oder andere dem Fachmann bekannte Hilfsstoffe enthalten. Die Verabreichung der beschriebenen Nukleinsäure gegebenenfalls in Form der oben näher beschriebenen Virusvektoren oder als Liposomenkomplexe bzw. Goldpartikelkomplex erfolgt üblicherweise topisch und lokal im Bereich der Wunde. Es ist auch möglich, das Polypeptid selbst mit geeigneten Zusatz- oder Hilfsstoffen, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., zu verabreichen, um die Wundheilung sofort und unmittelbar zu beeinflussen.

Die Therapie von Erkrankungen, beispielsweise Hauterkrankungen, und/oder bei der Wundheilung, kann mittels der erfindungsgemäßen Arzneimittel über orale Dosierungsformen, wie z.B. Tabletten oder Kapseln, über die Schleimhäute, zum Beispiel der Nase oder der Mundhöhle, oder in Form von unter die Haut implantierten Dispositorien erfolgen. Transdermale therapeutische Systeme (TTS) sind zum Beispiel aus den EP 0 944 398, EP 0 916 336, EP 0 889 723 oder EP 0 852 493 bekannt.

Die Therapie kann jedoch auch, z.B. durch Verbände, Pflaster, Kompressen oder Gele erfolgen, die die erfindungsgemäßen Arzneimittel enthalten. Diese Therapie ist beispielsweise bei der Behandlung von Hauterkrankungen und/oder bei der Wundheilung bevorzugt. So ist es möglich, die geeignete Zusatz- oder Hilfsstoffe, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., enthaltenden Arzneimittel topisch und lokal zu verabreichen, um die Wundheilung sofort und unmittelbar zu beeinflussen. Die Verabreichung der erfindungsgemäßen Arzneimittel kann weiterhin gegebenenfalls in Form von Liposomenkomplexen bzw. Goldpartikelkomplexen ebenfalls topisch und lokal im Bereich der Wunde erfolgen. Weiterhin kann die Behandlung mittels eines TTS erfolgen, das eine zeitlich gesteuerte Abgabe der erfindungsgemäßen Arzneimittel ermöglicht. Die Therapie unter Verwendung von Zellen, die mindestens eine ein erfindungsgemäßes Polypeptid oder Varianten davon kodierende Nukleinsäure oder Varianten davon exprimieren, kann beispielsweise mit autologen oder heterologen Zellen erfolgen. Bevorzugte Zellen sind Hautzellen, beispielsweise dermale oder epidermale Zellen, insbesondere Keratinozyten, Fibroblasten und Endothelzellen. Die Zellen können dann direkt oder gegebenfalls mit geeigneten Trägermaterialien auf betroffenes Gewebe, bevorzugt Haut, insbesondere in eine Hautwunde aufgebracht werden (US 5,980,888; WO 92/06179; EP 0242 270; WO 90/02796).

Beispiele von Erkrankungen der Hautzellen bzw. Hauterkrankungen im Sinne der vorliegenden Erfindung sind Psoriasis, Ekzeme, insbesondere atopisches Ekzeme, Akne, Urtikaria, Pigmentstörungen der Haut, insbesondere Vitiligo, "senile skin" und Störungen des Haarwachstums oder -metabolismus. Eine besonders bevorzugte Hauterkrankung ist Psoriasis.

Unter Wundheilung im Sinne der vorliegenden Erfindung ist der Heilungsprozeß einer mechanischen Verletzung der Haut zu verstehen, wie z.B. Schnittwunden, Schürfwunden oder das Aufreiben der Haut beispielsweise durch andauernde Belastung, beispielsweise Dekubitus oder nekrotische Prozesse, beispielsweise *Nekrobiosis lipoidica*.

Beispiele für gestörte Wundheilung bzw. krankhafte Störungen der Wundheilung im Sinne der vorliegenden Erfindung sind Wunden diabetischer Patienten und Alkoholiker, mit Organismen oder Viren infizierte Wunden, ischämische Wunden, Wunden von Patienten mit arteriellen Verschlußkrankheiten oder venöser Insuffizienz, und Narben, bevorzugt überschießende Narben, insbesondere Keloide. Besonders bevorzugte schlecht heilende Wunden sind diabetische, neuropathische, venöse, arterielle und Dekubitus-Ulzera, insbesondere venöse Ulzera.

Die vorliegende Erfindung betrifft weiterhin ein Verfahren zur Herstellung eines Diagnostikums zur Diagnose von Erkrankungen, beispielsweise Hauterkrankungen und/oder zur Diagnose bei der Wundheilung und/oder dessen krankhaften Störungen, das dadurch gekennzeichnet ist, daß mindestens eine Nukleinsäure, mindestens ein Polypeptid oder mindestens ein Antikörper, mindestens eine ein erfindungsgemäßes Polypeptid oder das Polypeptid kodierende Nukleinsäure exprimierende Zelle, wie vorangehend beschrieben, gegebenenfalls zusammen mit geeigneten Zusatz- und Hilfsstoffen, verwendet wird.

Beispielsweise kann gemäß der vorliegenden Erfindung anhand einer der beschriebenen Nukleinsäure ein Diagnostikum auf der Basis der Polymerasekettenreaktion (Beispiele 2-6, PCR-Diagnostik, z. B. gemäß EP 0 200 362) oder eines RNase-Protection-Assays (siehe z. B. Sambrook et al., supra Kapitel 7, Seite 7.71-7.78; Werner et al., 1992, Growth Factors and Receptors: A Practical Approach 175-197; Werner, 1998, Proc. Natl. Acad. Sci. U.S.A. 89: 6896-6900) hergestellt werden. Diese Tests beruhen auf der spezifischen Hybridisierung einer Nukleinsäure mit ihrem komplementären Gegenstrang, üblicherweise der entsprechenden mRNA oder ihrer cDNA. Die vorangehend beschriebenen Nukleinsäuren können hierbei auch modifiziert sein, wie z. B. in EP 0 063 879 offenbart. Vorzugsweise wird ein solches DNA-Fragment mittels geeigneter Reagenzien, z. B. radioaktiv mit α-P³²-dCTP oder nicht-radioaktiv mit Biotin oder Digoxigenin, nach allgemein bekannten Methoden markiert und mit isolierter RNA, die vorzugsweise vorher an geeignete Membranen aus z. B. Nitrocellulose oder Nylon gebunden wurde, inkubiert. Bei gleicher Menge an untersuchter RNA aus jeder Gewebeprobe kann somit die Menge an mRNA bestimmt werden, die spezifisch durch die Sonde markiert wurde. Alternativ kann die Bestimmung der mRNA Menge auch direkt in Gewebeschnitten mit Hilfe der in situ Hybridisierung (siehe z.B. Werner et al., 1992, Proc. Natl. Acad. Sci. U. S. A. 89:6896-900) erfolgen.

Mit Hilfe des erfindungsgemäßen Diagnostikums kann somit auch *in vitro* die Expressionsstärke des jeweiligen Gens in einer Gewebeprobe spezifisch gemessen werden, um beispielsweise eine Wundheilungsstörung oder dermatologische Erkrankungen sicher diagnostizieren zu können (Beispiele 2 bis 6). Insbesondere eignet sich ein solches Verfahren zur frühzeitigen Prognose von Störungen.

Ein weiterer Gegenstand der vorliegenden Erfindung betrifft ein Diagnostikum zur Diagnose von Erkrankungen, beispielsweise Hauterkrankungen und/oder zur Diagnose bei der Wundheilung und/oder dessen krankhafte Störungen, das mindestens eine erfindungsgemäße Nukleinsäure, mindestens ein erfindungsgemäßes Polypeptid, mindestens eine erfindungsgemäße Zelle, oder mindestens einen erfindungsgemäßen Antikörper oder Antikörperfragment, wie vorangehend beschrieben, gegebenenfalls zusammen mit geeigneten Zusatz- und Hilfsstoffen, umfaßt.

Ein bevorzugtes erfindungsgemäßes Diagnostikum enthält das beschriebene Polypeptid bzw. die oben näher beschriebenen immunogenen Teile davon. Das Polypeptid bzw. die Teile davon, die vorzugsweise an eine Festphase, z.B. aus Nitrocellulose oder Nylon, gebunden sind, können beispielsweise mit der zu untersuchenden Körperflüssigkeit, z.B. Wundsekret, *in vitro* in Berührung gebracht werden, um so beispielsweise mit Autoimmunantikörper reagieren zu können. Der Antikörper-Peptid-Komplex kann anschließend beispielsweise anhand markierter Antihuman-IgG- oder Antihuman-IgM-Antikörper nachgewiesen werden. Bei der Markierung handelt es sich beispielsweise um ein Enzym, wie Peroxidase, das eine Farbreaktion katalysiert. Die Anwesenheit und die Menge an anwesenden Autoimmunantikörper kann somit über die Farbreaktion leicht und schnell nachgewiesen werden.

Ein weiteres Diagnostikum, das Gegenstand der vorliegenden Erfindung ist, enthält die erfindungsgemäßen Antikörper selbst. Mit Hilfe dieser Antikörper kann beispielsweise eine Gewebeprobe leicht und schnell dahingehend untersucht werden, ob das betreffende Polypeptid in einer erhöhten Menge vorhanden ist, um dadurch einen Hinweis auf eine mögliche Erkrankung, beispielsweise Hauterkrankungen und Wundheilungsstörungen zu erhalten. In diesem Fall sind die erfindungsgemäßen Antikörper beispielsweise mit einem Enzym, wie oben bereits beschrieben, markiert, Der spezifische Antikörper-Peptid-Komplex kann dadurch leicht und schnell über eine enzymatische Farbreaktion nachgewiesen werden.

Ein weiteres erfindungsgemäßes Diagnostikum umfaßt eine Sonde, vorzugsweise eine DNA-Sonde, und/oder Primer. Dies eröffnet eine weitere Möglichkeit, die beschriebenen Nukleinsäuren, zum Beispiel durch die Isolierung aus einer geeigneten Genbank, beispielsweise aus einer wundspezifischen Genbank, anhand einer geeigneten Sonde zu erhalten (siehe z. B. J. Sambrook et al., 1989, Molecular Cloning. A Laboratory Manual 2^{nd} edn., Cold Spring Harbor Laboratory, Cold Spring Harbor, NY Kapitel 8 Seite 8.1 bis 8.81, Kapitel 9 Seite 9.47 bis 9.58 und Kapitel 10 Seite 10.1 bis 10.67).

Als Sonde eignen sich beispielsweise DNA- oder RNA-Fragmente mit einer Länge von ca. 100-1000 Nukleotiden, vorzugsweise mit einer Länge von ca. 200-500 Nukleotiden, insbesondere mit einer Länge von ca. 300-400 Nukleotiden deren Sequenz aus den Polypeptiden gemäß den SEQ ID Nr. 1 bis SEQ ID Nr. 4 des Sequenzprotokolls und/oder anhand der cDNA Sequenzen, die im Sequenzprotokoll gemäß einer SEQ ID Nr. 5 bis SEQ ID Nr. 8 aufgelistet sind, abgeleitet werden kann.

Alternativ können anhand der abgeleiteten Nukleinsäuresequenzen Oligonukleotide synthetisiert werden, die sich als Primer für eine Polymerase Kettenreaktion eignen. Mit diesen kann die vorangehend beschriebene Nukleinsäure oder Teile dieser aus cDNA, beispielsweise wundspezifischer cDNA oder Psoriasisspezifischer cDNA, amplifiziert und isoliert werden (Beispiele 2 bis 6). Als Primer eignen sich beispielsweise DNA-Fragmente mit einer Länge von ca. 10 bis 100 Nukleotiden, vorzugsweise mit einer Länge von ca. 15 bis 50 Nukleotiden, insbesondere mit einer Länge von 20 bis 30 Nukleotiden deren Sequenz aus den Polypeptiden gemäß den SEQ ID Nr. 1 bis SEQ ID Nr. 4 des Sequenzprotokolls und/oder anhand der cDNA Sequenzen, die im Sequenzprotokolls gemäß einer SEQ ID Nr. 5 bis SEQ ID Nr. 8 aufgelistet sind, abgeleitet werden kann.

Ein weiterer Gegenstand der Erfindung betrifft die Verwendung des erfindungsgemäßen Diagnostikums zur Diagnose von Erkrankungen, beispielsweise Hauterkrankungen, und/oder zur Diagnose bei der Wundheilung und/oder deren krankhafte Störungen.

Ein weiterer Gegenstand der Erfindung betrifft ein Verfahren zur Herstellung eines Tests zur Auffindung von pharmakologisch aktiven Substanzen, dadurch gekennzeichnet, daß mindestens eine erfindungsgemäße Nukleinsäure, mindestens ein erfindungsgemäßes Polypeptids, mindestens ein erfindungsgemäßes Fusionsprotein mindestens ein erfindungsgemäßer Antikörper oder Antikörperfragment, mindestens eine erfindungsgemäße Zelle, oder mindestens ein erfindungsgemäßes nichtmenschliches transgenes Säugetier, gegebenenfalls zusammen mit geeigneten Zusatz- und Hilfsstoffen, zur Herstellung des Tests verwendet wird.

Die Erfindung umfaßt weiterhin einen erfindungsgemäß hergestellten Test zur Identifizierung von pharmakologisch aktive Substanzender mindestens eine erfindungsgemäße Nukleinsäure, mindestens ein erfindungsgemäßes Polypeptid mindestens einen erfindungsgemäßen Antikörper oder Antikörperfragment, oder mindestens eine erfindungsgemäße Zelle gemäß der vorliegenden Erfindung, gegebenenfalls zusammen mit geeigneten Zusatz- und Hilfsstoffen, umfaßt. Dieser Test kann zur Identifizierung von pharmakologisch aktive Substanzen in Zusammenhang mit Erkrankungen, insbesondere Hauterkrankungen, und/oder in Zusammenhang mit Wundheilung und/oder deren krankhaften Störungen eingesetzt werden.

In einer bevorzugten Ausführungsform wird für den Test mindestens eine, ein erfindungsgemäßes Polypeptid oder Varianten davon oder dieses kodierende Nukleinsäure oder Varianten davon exprimierende Zelle verwendet.

Weiterhin umfaßt die Erfindung einen Test, wobei mindestens ein erfindungsgemäßes Polypeptid, und/oder mindestens eine erfindungsgemäße Nukleinsäure, und/oder mindestens ein erfindungsgemäßer Antikörper oder Antikörperfragment, und/oder mindestens eine erfindungsgemäße Zelle, an eine Festphase gebunden wird. Dieser Test kann zur Identifizierung von pharmakologisch aktive Substanzen in Zusammenhang mit Erkrankungen, insbesondere Hauterkrankungen, und/oder in Zusammenhang mit Wundheilung eingesetzt werden.

Ein geeignetes System läßt sich beispielsweise durch die stabile Transformation von Zellen, beispielsweise epidermalen bzw. dermalen Zellen, mit Expressionsvektoren, die selektierbare Markergene und die beschriebenen Nukleinsäuren enthalten, herstellen. Bei diesem Verfahren wird die Expression der beschriebenen Nukleinsäuren in den Zellen so verändert, daß sie der pathologisch gestörten Expression *in vivo* entspricht. Auch anti-sense Oligonukleotide, die die beschriebenen Nukleinsäure enthalten, können zu diesem Zweck eingesetzt werden. Von besonderem Vorteil für diese Systeme ist es daher, das Expressionsverhalten der Gene bei gestörten regenerativen Prozessen, wie in dieser Anmeldung offengelegt, zu kennen. Oft kann so das pathologische Verhalten der Zellen in vitro nachgeahmt werden und es können Substanzen gesucht werden, die das normale Verhalten der Zellen wieder herstellen und die ein therapeutisches Potential besitzen.

Für diese Testsysteme eignen sich z.B. HaCaT-Zellen, die allgemein erhältlich sind, und der Expressionsvektor pCMV4 (Anderson et al., 1989, J. Biol. Chem. 264:8222-9). Die vorangehend beschriebene Nukleinsäure kann dabei sowohl in sense als auch in anti-sense Orientierung in die Expressionsvektoren integriert werden, so daß die funktionelle Konzentration an mRNA der entsprechenden Gene in den Zellen entweder erhöht, oder durch Hybridisierung mit der antisense-RNA erniedrigt wird. Nach der Transformation und Selektion stabiler Transformanden zeigen die Zellen in Kultur im allgemeinen ein verändertes Proliferations- , Migrations-, und/oder Differenzierungsverhalten im Vergleich zu Kontrollzellen. Dieses Verhalten *in vitro* ist häufig mit der Funktion der entsprechenden Gene bei regenerativen Prozessen im Organismus korreliert (Yu et al., 1997, Arch. Dermatol. Res. 289:352-9; Mils er al., 1997, Oncogene 14: 15555-61; Charvat et al., 1998, Exp Dermatol 7: 184-90; Werner, 1998, Cytokine Growth Factor Rev. 9:153-65; Mythily et al., 1999, J. Gen. Virol. 80:1707-13) und läßt sich mit einfachen und schnell durchzuführenden Tests nachweisen, so daß man darauf basierend Testsysteme für pharmakologisch aktive Substanzen aufbauen kann. So läßt sich das Proliferationsverhalten von Zellen sehr schnell durch z.B. den Einbau von markierten Nukleotiden in die DNA der Zellen (siehe z.B. Savino und Dardenne, 1985, J. Immunol. Methods 85:221-6; Perros und Weightman, 1991, Cell Prolif. 24:517-23; de Fries und Mitsuhashi, 1995, J. Clin. Lab. Anal. 9:89-95), durch Anfärbung der Zellen mit spezifischen Farbstoffen (Schulz et al., 1994, J. Immunol. Methods 167:1-13) oder über immunologische Verfahren (Frahm et al., 1998, J. Immunol. Methods 211:43-50) nachweisen. Die Migration läßt sich einfach durch den "Migration Index" Test (Charvat et al., supra) und vergleichbare Testsysteme (Benestad et al., 1987, Cell Tissue Kinet. 20:109-19, Junger et al., 1993, J. Immunol. Methods 160:73-9) nachweisen. Als Differenzierungsmarker eignen sich z.B. Keratin 6, 10 und 14 sowie Loricrin und Involucrin (Rosenthal et al., 1992, J. Invest. Dermatol. 98:343-50), deren Expression z.B. über allgemein erhältliche Antikörper leicht nachzuweisen ist.

Ein anderes geeignetes Testsystem basiert auf der Identifikation von Interaktionen mit dem sogenannten "Two-Hybrid System" (Fields und Sternglanz, 1994, Trends in Genetics, 10, 286-292; Colas und Brent, 1998 TIBTECH, 16, 355-363). Bei diesem Test werden Zellen mit Expressionsvektoren transformiert, die Fusionsproteine aus dem vorangehend beschriebenen Polypeptid und einer DNA-Bindungsdomäne eines Transkriptionsfaktors wie beispielsweise Gal4 oder LexA exprimieren. Die transformierten Zellen enthalten außerdem ein Reportergen, dessen Promotor Bindungsstellen für die entsprechende DNA Bindungsdomäne enthalten. Durch Transformation eines weiteren Expressionsvektors, der ein zweites Fusionsprotein aus einem bekannten bzw. unbekannten Polypeptid mit einer Aktivierungsdomäne, beispielsweise von Gal4 oder Herpes simplex Virus VP16, exprimiert, kann die Expression des Reportergens stark gesteigert werden, wenn das zweite Fusionsprotein mit dem vorangehend beschriebenen Polypeptid interagiert. Diese Expressionssteigerung kann man ausnutzen, um neue pharmakologisch aktive Substanzen zu identifizieren, beispielsweise indem man zur Konstruktion des zweiten Fusionsproteins eine cDNA-Bibliothek aus sich regenerierenden Gewebe herstellt. Zudem läßt sich dieses Testsystem zum Screening von Substanzen ausnutzen, die eine Interaktion zwischen dem vorangehend beschriebenen Polypeptid und einem Interaktor inhibieren. Solche Substanzen verringern die Expression des Reportergens in Zellen, die Fusionsproteine des vorangehend beschriebenen Polypeptids und der pharmakologisch aktiven Substanzen exprimieren (Vidal und Endoh, 1999, Trends in Biotechnology, 17:374-81). So lassen sich schnell neue Wirkstoffe identifizieren, die zur Therapie von Störungen beispielsweise regenerativer Prozesse eingesetzt werden können.

Weitere zell-basierte Testsysteme, die sich insbesondere zur Analyse von GPCR Polypeptiden eignen sind beispielsweise in Marchese et al. (1999, Trends in Pharmacol. Sci., 20: 370-375) zusammengestellt und umfassen sogenannte Liganden-Screening Assays insbesondere in Zellen mit niedrigem endogenen GPCR Hintergrund-Expression oder gut meßbaren G-Protein Aktivierungsprozessen oder beidem. Beispielsweise kann der Pheromonrezeptor in Hefezellen durch ein erfindungsgemäßes Polypeptid ersetzt werden. Der Einfluß von Testsubstanzen auf den Rezeptor kann durch Modulation der Histidinsynthese bzw. des Wachstums in Histidin-freiem Medium gemessen werden. Weiterhin kann in mit erfindungsgemäßen Nukleinsäuren transfizierten Zellen untersucht werden, ob Testsubstanzen die Translokation eines detektierbaren Arrestins, beispielsweise eines Arrestin-GFP-Fusionsproteins vermitteln. Weiterhin kann untersucht werden, ob Testsubstanzen die GPCR-vermittelte Dispersion oder Aggregation von *Xenopus laevis* Melanophoren vermitteln. Ein weiteres Testsystem bedient sich des universalen Adapter G-Proteins Galpha16, der zur Mobilisierung von Ca²⁺ führt. Weitere Screening Testsysteme sind in Lerner et al., supra; WO 96/41169; US 5,482,835; WO 99/06535; EP 0939902; WO 99/66326; WO 98/34948; EP 0863214; US 5,882,944 und US 5,891,646 zusammengestellt.

Eine weitere bevorzugte Ausführungsform besteht ein Test zur Identifizierung von pharmakologisch aktiven Substanzen darin, daß pharmakologisch aktive Substanzen getestet werden ob sie einen Effekt auf ein erfindungsgemäßes nichtmenschliches transgenes Säugetier im Zusammenhang mit Erkrankungen, insbesondere Hauterkrankungen und/oder Wundheilung und/oder deren krankhaften Störungen haben. Die erfindungsgemäßen nichtmenschlichen transgenen Säugetiere können auf veränderte Eigenschaften, beispielsweise auf veränderte Wundheilung oder epidermale Proliferation, insbesondere stimulierte epidermale Proliferation oder veränderte Inflammation hin getetstet werden. Mögliche pharmakologisch aktive Substanzen können nun dahingehend getestet werden, ob sie diese veränderten Eigenschaften der Tiere verändern, beispielsweise die Wundheilung ändern, insbesondere die Wundheilung stimulieren oder ob sie die veränderte, beispielsweise inhibierte oder stimulierte, epidermale Proliferation oder Inflammation verändern, beispielsweise aktivieren oder inhibieren.

In einer weiteren bevorzugten Ausführungsform der Erfindung besteht ein Test zur Identifizierung pharmakologisch aktiver Substanzen darin, daß pharmakologisch aktiven Substanzen getestet werden ob sie die Expression mindestens einer erfindungsgemäßen Nukleinsäure beeinflussen.

Assays zur Identifikation von pharmakologischen Substanzen, die die Expression von Genen beeinflussen, sind dem Fachmann allgemein bekannt (siehe beispielsweise Sivaraja et al., 2001, US 6,183,956).

So können Zellen, die erfindungsgemäße Nukleinsäuren exprimieren, beispielsweise HeLa Zellen, als Testsystem zur Analyse der Genexpression *in vitro* kultiviert werden, wobei Hautzellen, insbesondere Keratinozyten, Fibroblasten oder Endothelzellen zu bevorzugen sind. Ein mögliches Testsystem stellt dabei die menschliche Keratinozytenzellinie HaCaT da, die allgemein erhältlich ist.

Die Analyse der Genexpression erfolgt beispielsweise auf der Ebene der mRNA oder der Proteine. Dabei wird die Menge an erfindungsgemäße Nukleinsäure oder Protein nach Zugabe einer oder mehrerer Substanzen zur Zellkultur gemessen und mit der entsprechenden Menge in einer Kontrollkultur verglichen. Dies geschieht beispielsweise mit Hilfe einer Hybridisierung einer anti-sense Sonde, mit der im Lysat der Zellen enthaltene mRNA von die erfindungsgemäßen Targetgenen nachgewiesen werden kann. Die Hybridisierung kann beispielsweise über die Bindung eines spezifischen Antikörpers an den mRNA-Sonden Komplex quantifiziert werden (siehe Stuart and Frank, 1998, US 4,732,847). Dabei ist es möglich, die Analyse im Hochdurchsatzverfahren durchzuführen und sehr viele Substanzen auf ihre Eignung als Modulator der Expression von erfindungsgemäßen Nukleinsäuren zu analysieren (Sivaraja et al., 2001, US 6,183,956). Die zu analysierenden Substanzen können dabei aus Substanzbibliotheken (siehe z.B. DE19816414, DE19619373) entnommen werden, die mehrere tausend, oft sehr heterogene Substanzen enthalten. Alternativ kann zunächst die gesamt RNA oder mRNA aus Zellen isoliert werden, und anschließend die absolute Menge oder der relative Anteil der mRNA von erfindungsgemäßen Targetgenen beispielsweise mit Hilfe der quantitativen RT-PCR (siehe EP 0 200 362; Wittwer et al., 1997, BioTechniques 22:130-8; Morrison et al., 1998, BioTechniques 24: 954-62) oder des RNAse Protection Assays (siehe z. B. Sambrook et al., 1989, Molecular cloning: A Laboratory Manual, Cold Spring Harbor, Cold Spring Harbor Laboratory Press, New York, Kapitel 7; EP 0 063 879) bestimmt werden. Eine weitere Möglichkeit stellt die Analyse der Proteinmenge im Zelllysat mit Antikörpern dar, die erfindungsgemäße Polypeptide verwenden, erkennen. Hier kann die Quantifizierung beispielsweise mit Hilfe eines ELISAs oder eines Western-Blots erfolgen, die allgemein bekannt sind. Um die Spezifität der Substanzen auf die Expression erfindungsgemäßer Nukleinsäuren zu bestimmen, kann der Einfluß von Substanzen auf die Targetgen Expression mit dem Einfluß auf die Expression andere Gene, wie beispielsweise Cyclophilin oder Gene des Stoffwechsels wie GAPDH, verglichen werden. Dies kann entweder in separaten Analysen erfolgen, oder parallel zur Analyse der erfindungsgemäßen Nukleinsäuren.

Weiterhin kann ein Testsystem darauf beruhen, daß erfindungsgemäße Polypeptide oder funktionelle Varianten davon und/oder diese kodierende Nukleinsäuren oder Varianten davon, und/oder gegen erfindungsgemäße Polypeptide oder funktionelle Varianten davon gerichtete Antikörper oder Antikörperfragmente oder erfindungsgemäße Polypeptide oder Varianten davon oder diese kodierende Nukleinsäuren oder Varianten davon exprimierende Zellen an eine Festphase gebunden werden und Substanzen auf Interaktion, beispielsweise Bindung oder Änderung der Konformation getestet werden. Geeignete Systeme wie die Affinitätschromatographie und Fluoreszenzspektroskopie sind dem Fachmann bekannt.

Die Festphasen-gebundenen erfindungsgemäßen Polypeptide oder funktionellen Varianten davon oder diese kodierenden Nukleinsäuren oder Varianten davon, oder erfindungsgemäße Antikörper oder ein Antikörperfragmente oder erfindungsgemäße Polypeptide oder Varianten davon oder diese kodierende Nukleinsäuren oder Varianten davon exprimierende Zellen können auch Teil eines Arrays sein.

Die Erfindung umfaßt ferner ein auf einem Trägermaterial fixiertes Array, bei dem mindestens ein erfindungsgemäßes GPCR-Polypeptid und oder erfindungsgemäße Fusionsproteine, und/oder erfindungsgemäße Nukleinsäure und/oder mindestens ein erfindungsgemäßer Antikörper oder Antikörperfragment und/oder mindestens eine, ein erfindungsgemäßes Polypeptid oder eine Variante davon oder eine diese kodierende Nukleinsäure oder eine Variante davon exprimierende Zelle auf einem Trägermaterial fixiert wird, eingesetzt werden. Diese Arrays können zur Analyse im Zusammenhang mit Erkrankungen von Hautzellen und/oder von Wundheilung und/oder deren krankhaften Störungen eingesetzt werden.

Die Erfindung bezieht sich weiterhin auch Verfahren zur Herstellung eines auf einem Trägermaterial fixierten Arrays, wobei mindestens ein erfindungsgemäßes Polypeptid, mindestens eine das Polypeptid kodierende Nukleinsäure, und/oder mindestens einen Antikörper oder Antikörperfragment, das gegen das Polypeptid gerichtet ist, und/oder mindestens eine, ein erfindungsgemäßes Polypeptid oder eine Variante davon oder eine diese kodierende Nukleinsäure oder eine Variante davon exprimierende Zelle auf einem Trägermaterial fixiert wird.

Verfahren zur Herstellung von solchen Arrays sind zum Beispiel aus der US 5,744,305 mittels Festphasenchemie und photolabilen Schutzgruppen bekannt. Diese Arrays können ebenfalls mit Substanzen oder Substanzbibliotheken in Kontakt gebracht werden und auf Interaktion, beispielsweise Bindung oder Konformationsänderung, getestet werden.

So kann beispielsweise eine zu testende Substanz einen detektierbaren Marker enthalten, beispielsweise kann die Substanz radioaktiv markiert, Fluoreszenz-markiert oder Lumineszenz-markiert sein. Weiterhin können Substanzen an Proteine gekoppelt werden, die eine indirekte Detektion erlauben, beispielsweise über enzymatische Katalyse mittels eines Peroxidase-Assays mit einem chromogenen Substrat oder durch Bindung eines detektierbaren Antikörpers. Änderungen der Konformation eines erfindungsgemäßen Polypeptide durch Interaktion mit einer Testsubstanz können beispielsweise durch Änderung der Fluoreszenz eines endogenen Tryptophan-Restes im Polypeptid nachgewiesen werden.

Die mit Hilfe der erfindungsgemäßen Testverfahren identifizierten pharmakologisch aktiven Substanzen können, gegebenenfalls kombiniert oder zusammen mit geeigneten Zusatz- und/oder Hilfsstoffen, zur Herstellung eines Diagnostikums oder Arzneimittels zur Diagnose, Prävention und/oder Behandlung von Erkrankungen von Hautzellen, und/oder zur Diagnose und/oder Behandlung von Wundheilung und/oder deren krankhaften Störungen eingesetzt werden.

Pharmakologisch aktive Substanzen der vorangehend beschriebenen Polypeptide können auch Nukleinsäuren sein, die über Selektionsverfahren, wie beispielsweise SELEX (siehe Jayasena, 1999, Clin. Chem. 45:1628-50; Klug und Famulok, 1994, M. Mol. Biol. Rep. 20:97-107; Toole et al., 1996, US 5,582,981) isoliert wereden. Im SELEX-Verfahren werden typischerweise aus einem großen Pool unterschiedlicher, einzelsträngige RNA Moleküle durch wiederholte Amplifikation und Selektion diejenigen Moleküle isoliert, die an ein Polypeptid mit hoher Affinität binden (Aptamere). Aptamere können auch in ihrer spiegelbildlichen Form, beispielsweise als L-Ribonukleotid, synthetisiert und selektioniert werden (Nolte et al., 1996, Nat. Biotechnol. 14:1116-9; Klussmann et al., 1996, Nat. Biotechnol. 14:1112-5). So isolierte Formen haben den Vorteil, das sie nicht von natürlich vorkommenden Ribonukleasen abgebaut werden und daher größere Stabilität besitzen.

Die identifizierten pharmakologisch aktiven Substanzen können, gegebenenfalls kombiniert oder zusammen mit geeigneten Zusatz- und/oder Hilfsstoffen, zur Herstellung eines Diagnostikums oder eines Arzneimittels zur Prävention, Behandlung und/oder Diagnose von Erkrankungen, beispielsweise Erkrankungen von Hautzellen und/oder zur Diagnose und/oder Behandlung bei der Wundheilung und/oder deren krankhaften Störungen eingesetzt werden. Die pharmakologisch aktiven Substanzen können beispielsweise anorganische oder organische Moleküle, beispielsweise Nukleinsäuren oder Nukleinsäurenanaloga, Peptide oder Proteine, insbesondere Antikörper, funktionelle Varianten erfindungsgemäß verwendbarer Polypeptide oder diese kodierende Nukleinsäuren oder Liganden erfindungsgemäß verwendbarer Polypeptide sein. Beispiele pharmakologisch aktiver Substanzen sind weiterhin organische Moleküle, die Substanzbibliotheken entstammen, die auf ihre pharmakologische Aktivität hin überprüft wurden.

Die Verwendung von Nukleinsäuren als Diagnostika kann beispielsweise auf der Basis der Polymerasekettenreaktion wie oben beschrieben erfolgen. Die Verwendung von Nukleinsäuren als Arzneimittel kann beispielsweise gentherapeutisch unter Verwendung eines gentherapeutisch wirksamen Vektors oder mittels Applikation von antisense Nukleotids wie beschrieben erfolgen.

Die Verwendung von anderen organischen oder anorganischen pharmakologisch aktiven Substanzen als Arzneimittel kann beispielsweise durch Applikation wie oben beschrieben erfolgen. Die Verwendung beispielsweise von Antikörpern als Diagnostika kann wie oben beschrieben mittels immunologischer Techniken erfolgen, beispielsweise unter Verwendung von Antikörpern, die mit einem Enzym markiert sind. Der spezifische Antikörper-Peptid-Komplex kann dadurch leicht und ebenso schnell über eine enzymatische Farbreaktion nachgewiesen werden.

Um pharmakologisch aktive Substanzen als Diagnostikum zu verwenden, können die Substanzen einen detektierbaren Marker enthalten, beispielsweise kann die Substanz radioaktiv markiert, Fluoreszenz-markiert oder Lumineszenz-markiert sein. Weiterhin können Substanzen an Enzyme gekoppelt werden, die eine indirekte Detektion erlauben, beispielsweise wie oben beschrieben über enzymatische Katalyse mittels eines Peroxidase-Assays mit einem chromogenen Substrat oder durch Bindung eines markierten oder detektierbaren Antikörpers. Die Substanzen können dann mit einer zu untersuchenden Probe in Kontakt gebracht werden und so die Menge eines erfindungsgemäß verwendbaren Polypeptids oder einer funktionellen Variante davon oder dieses kodierende Nukleinsäure oder einer Variante davon, oder einer ein erfindungsgemäß verwendbares Polypeptid oder einer funktionellen Variante davon oder einer dieses kodierende Nukleinsäure oder eine Variante davon exprimierenden Zelle, oder eines gegen ein erfindungsgemäß verwendbares Polypeptid gerichteten Antikörpers oder eines Antikörperfragments in der Probe bestimmt werden. Die Ergebnisse dieser Probe, die einem zu untersuchenden Organismus entstammt, kann dann verglichen werden mit dem Ergebnis einer Probe, die einem gesunden oder kranken Organismus entstammt.

Ein weiterer Gegenstand der Erfindung ist ein Verfahren zur Herstellung eines auf einem Trägermaterial fixierten Arrays zur Analyse in Zusammenhang mit Erkrankungen, beispielsweise Hauterkrankungen, und/oder in Zusammenhang mit Wundheilung, bei dem mindestens eine erfindungsgemäße Nukleinsäure, mindestens ein erfindungsgemäßes Polypeptid,mindestens ein erfindungsgemäßer Antikörper oder Antikörperfragment, oder mindestens eine, ein erfindungsgemäßes Polypeptid oder eine Variante davon oder eine diese kodierende Nukleinsäure oder eine Variante davon exprimierende Zelle wie vorangehend beschrieben, zur Herstellung verwendet wird. Diese Arrays können zur Analyse im Zusammenhang mit Erkrankungen, beispielsweise Hauterkrankungen und/oder von Wundheilung und/oder deren krankhaften Störungen eingesetzt werden.

Verfahren zur Herstellung von solchen Arrays sind zum Beispiel aus der US 5,744,305 mittels Festphasenchemie und photolabilen Schutzgruppen bekannt.

Ein weiterer Gegenstand der Erfindung ist ein auf einem Trägermaterial fixiertes Array zur Analyse in Zusammenhang mit Erkrankungen, beispielsweise Hauterkrankungen, und/oder in Zusammenhang mit Wundheilung, das dadurch gekennzeichnet ist, daß es mindestens eine Nukleinsäure und/oder mindestens ein Polypeptid und/oder mindestens einen Antikörper oder Antikörperfragment, und/oder mindestens eine, ein erfindungsgemäßes Polypeptid oder eine Variante davon oder eine diese kodierende Nukleinsäure oder eine Variante davon exprimierende Zelle wie vorangehend beschrieben, umfaßt.

Die Erfindung soll nun im folgenden anhand der Figuren und Beispiele weiter verdeutlicht werden, ohne daß die Erfindung hierauf eingeschränkt wird.

### Beschreibung der Tabellen, Figuren und Sequenzen:

- Figur 1:: Autoradiogramme von Hybridisierungen von Membranen mit gleichem Muster von aufgebrachten cDNA-Fragmenten mit zwei verschiedenen Sonden. Die cDNA Fragmente entstammten alle einer wundspezifischen, subtraktiven cDNA-Bibliothek, die für solche cDNAs angereichert war, die in normal heilendem Wundgewebe stärker im Vergleich zur intakten Haut exprimiert wurden. Beide Sonden wurden aus cDNAs hergestellt, die aus subtraktiven Hybridisierungen stammten. A: hautspezifische Sonde (Subtraktion intakte Haut versus normal heilende Wunde) B: wundspezifische Sonde (Subtraktion normal heilende Wunde versus intakte Haut). Die Positionen der SW1368 cDNAs (jeweils doppelt aufgetragen) sind mit Pfeilen gekennzeichnet.
- Figur 2:: Tabellarische Aufstellung der veränderten Expression der wundheilungsrelevanten murinen SW1368 und SW1695 Gene in normal heilenden Wunden und in mit Dexamethason-behandelten, schlecht heilenden Wunden von 10 Wochen alten BALB/c Mäusen als auch in Wunden von jungen (4 Wochen) und alten (12 Monate) BALB/c Mäusen.
- Figur 3:: Tabellarische Aufstellung der veränderten Expression der wundheilungsrelevanten murinen SW1368 und SW1695 Gene in Mäusen mit Diabetes und in Kontrolltieren.

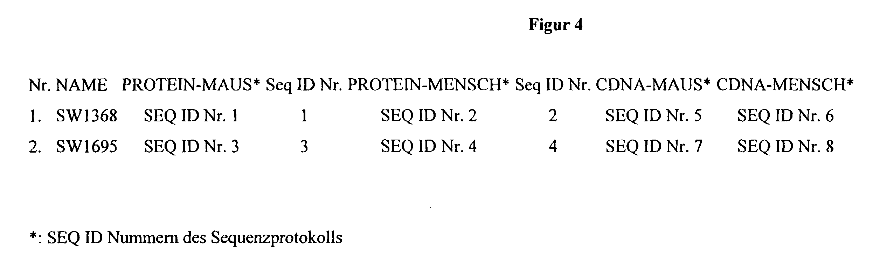

- Figur 4:: Tabellarische Übersicht über die bei der Analyse der Genexpression während des Wundheilungsprozesses identifizierten Polypeptidsequenzen der Genfamilie und ihre cDNAs.
- Figur 5:: Vergleich der Polypeptidsequenz der identifizierten Proteine von SW1368 aus Maus und Mensch. Exakte Übereinstimmungen der Maus Sequenz von SW1368 mit der humanen Sequenz von SW1368 sind markiert.
- Figur 6:: Vergleich der Polypeptidsequenz der identifizierten Proteine von SW1695 aus Maus und Mensch. Exakte Übereinstimmungen der Maus Sequenz von SW1695 mit der humanen Sequenz von SW1695 sind markiert.
- Figur 7:: Kinetik der Expression von humanem SW1368 während der Wundheilung im Menschen.
- Figur 8:: Analyse der relativen Expression von humanem SW1368 und SW1695 in Wundgrund bzw. Wundrand relativ zu intakter Haut von *Ulcus venosum-*Patienten.

SEQ ID Nr. 1 bis SEQ ID Nr. 8 zeigen die erfindungsgemäßen Polypeptid- oder cDNA-Sequenzen aus Mensch oder Maus.

SEQ ID Nr. 9 bis SEQ ID Nr. 14 und SEQ ID Nr. 16 bis SEQ ID Nr. 21 zeigen DNA-Sequenzen von Oligonukleotiden, die für die Versuche der vorliegenden Erfindung verwendet wurden.

SEQ ID Nr. 15 zeigt die Teilsequenz der erfindungsgemäß verwendeten cDNA gemäß einer SEQ ID Nr. 1, die durch Sequenzieren eines als reguliert identifizierten Klons in der "Reverse Northern Blot Analyse" ermittelt wurde.

### Beispiele

### Beispiel 1: Anreicherung von wundrelevanter cDNA mittels subtraktiver Hybridisierung und Identifizierung von SW1368 als wundrelevantes Gen

Aus intakter Haut und aus Wundgewebe (Verwundung am Rücken 1 Tag vor Gewebeentnahme durch Scherenschnitt) von BALB/c Mäusen wurde durch Standardmethoden (Chomczynski und Sacchi, 1987, Anal. Biochem. 162: 156-159, Chomczynski und Mackey, 1995, Anal. Biochem. 225: 163-164) Gesamt-RNA isoliert. Die RNAs wurden dann mit Hilfe einer reversen Transkriptase in cDNA umgeschrieben. Die cDNA Synthese erfolgte mit dem "SMART PCR cDNA Synthesis Kit" der Firma Clontech Laboratories GmbH, Heidelberg, nach Anweisungen des entsprechenden Manuals.

Um diejenigen cDNAs zu identifizieren, die mit unterschiedlicher Häufigkeit in den cDNA Pools vorkamen, wurde eine subtraktive Hybridisierung (Diatchenko et al., 1996, Proc. Natl. Acad. Sci. U.S.A. 93: 6025-30) durchgeführt. Dies erfolgte mit dem "PCR-Select cDNA Subtraction Kit" der Firma Clontech Laboratories GmbH, Heidelberg, nach Anweisungen des entsprechenden Manuals, wobei die Abtrennung überschüssiger Oligonukleotide nach der cDNA Synthese über Agarose-Gelelekrophorese erfolgte. Es wurden zwei für wundrelevante Gene angereicherte cDNA Pools angelegt, wobei ein Pool für cDNA Fragmente angereichert war, die im normal heilenden Wundgewebe im Vergleich zu intakter Haut stärker exprimiert sind ("wundspezifischer cDNA Pool"), während der andere Pool an cDNA Fragmenten angereichert war, die in intakter Haut im Vergleich zu normal heilendem Wundgewebe stärker exprimiert sind ("hautspezifischer cDNA Pool").

Um diejenigen Gene zu identifizieren, die in den wundheilungsrelevanten cDNA-Pools enthalten waren, wurde das Vorhandensein der entsprechenden cDNAs in den Pools im "Reverse Northern Blot" analysiert. Hier werden cDNA-Fragmente auf Membranen in Form von Arrays von vielen verschiedenen cDNAs fixiert, und mit einem komplexen Gemisch radioaktiv markierter cDNA hybridisiert (Sambrook et al., 1989, Molecular Cloning: A Laboratory Manual, Cold Spring Harbor, Cold Spring Harbor Laboratory Press, New York, Kapitel 9 Seite 9.47 bis 9.58 und kapitel 10 Seite 10.38 bis 10.50; Anderson and Young: Quantitative Filter ; in: Nucleic Acids Hybridisation, A Practical Approach, 1985, Eds. Hames and Higgins, IRL Press Ltd.; Oxford, Kapitel 4, Seite 73 bis 112). Auf den in diesem Beispiel verwendeten Membranen waren cDNA-Fragmente fixiert, die einer wundspezifischen, subtraktiven cDNA-Bibliothek entstammen, die für solche cDNAs angereichert war, die in normal heilendem Wundgewebe stärker im Vergleich zur intakten Haut exprimiert wurden.
Zur Herstellung geeigneter Hybridisierungssonden wurden die subtrahierten cDNA Pools mit der Restriktionsendonuklease RsaI behandelt und über Agarosegelelektrophorese gereinigt (Sambrook et al., supra, Kapitel 6, Seite 6.1 bis 6.35), um die cDNA- Synthese- und Amplifikationsprimer (siehe Manual "PCR-Select cDNA Subtraction Kit", Clonetech) abzutrennen. Die cDNAs wurde dann mit der "random-hexamer priming" Methode (Feinberg und Vogelstein, 1983, Anal. Biochem. 132:6-13) radioaktiv markiert, um Hybridisierungssonden herzustellen.

Die Membran wurde für 30 min bei 65 °C in 25 ml Hybridisierungslösung vorinkubiert (25 mM Natriumphosphat, pH = 7,5, 125 mM NaCl, 7% SDS). Die Hybridisierungssonde wurde 10 min bei 100°C denaturiert, anschließend auf Eis abgekühlt, ca. 100 CPM ("counts per minute") pro ml zur Hybridisierungslösung gegeben und die Hybridisierung für 16 Stunden bei 65 °C im Hybridisierungsofen durchgeführt. Danach wurde die Membran zweimal für 10 min mit der Hybridisierungslösung ohne Sonde bei 65 °C gewaschen. Anschließend wurde die Membran mehrfach für jeweils 10 min in Waschlösung (2,5 mM Natriumphosphat, pH = 7,5, 12,5 mM NaCl, 0,7% SDS) bei 65°C gewaschen, bis in der abgegossenen Lösung keine Aktivität mehr nachgewiesen werden konnte. Die radioaktiven Signale wurden mit einem Phosphoimager (BioRad, Quantitiy One®) und nachfolgender Analyse mittels Array Vision 4.0 (Imaging Research Inc.) ausgewertet. Dabei wurde eine Maske definiert, durch die Länge und Breite der Felder als auch der Durchmesser der Spotpositionen bestimmt wurde. Die Autoradiographie mit überlagerter Maske ist in Figur 1 dargestellt. Die Normierung der Signalintensitäten erfolgte über die Auswertung der Positivkontrolle, die aus LPHR cDNA aus Hefe bestand. Diese DNA wurde an definierten Positionen des Arrays gespottet und durch Zugabe einer geeigneten Sonde zu der Hybridisierungssonde des wundspezifischen bzw. des hautspezifischen cDNA-Pools quantifiziert. Es wurden diejenigen cDNAs ausgewählt, die mit den verwendeten Sonden unterschiedliche normierte Signalintensitäten ergaben. Dabei ergab sich an den Positionen von SW1368 auf der Membran eine deutlich stärkere Signalintensität mit der Hybridisierungssonde des wundspezifischen cDNA Pools (Figur 1B) im Vergleich zum hautspezifischen cDNA Pool (Figur 1A). Die Sequenzierung des Klons (Seq ID Nr. 15) und Analyse der Sequenz von 653 Basenpaar Länge ergaben, daß es sich um einen bislang unbekannten GPCR handelt.

### Beispiel 2: Verifikation des Expressionsmusters von SW1368 mittels "real time quantitative RTPCR"

Eine Verifikation der differentiellen Expression der vorangehend beschriebenen Nukleinsäuren als auch die Untersuchung weiterer Wundheilungszustände erfolgte über eine real-time RTPCR im ABI Prism 7700 Sequence Detection System (PE Applied Biosystems). Das Gerät war mit der ABI Prism 7200/7700 SDS-Software Version 1.6.3 (1998) ausgestattet. Der Nachweis von PCR Produkten erfolgte während der Amplifikation der cDNA mit Hilfe des Farbstoffs SYBR Green 1, dessen Fluoreszenz durch Bindung an doppelsträngige DNA stark erhöht wird (Karlsen et al. 1995, J. Virol. Methods. 55: 153-6; Wittwer et al., 1997, BioTechniques 22:130-8, Morrison et al., 1998, BioTechniques 24: 954-62). Basis fiir die Quantifizierung ist der PCR Zyklus ("threshold cycle", CT -Wert), der erreicht ist, wenn das Fluoreszenzsignal einen definierten Schwellenwert übersteigt. Die Auswertung erfolgt über die Δ-CT-Methode (User Bulletin #2, Relative Quantification of Gene Expression, PE Applied Biosystems, 1997). Die Abundanzen der cDNAs wurden relativ zu einer endogenen Referenz (GAPDH) bestimmt. Die Ergebnisse sind in Figuren 2 und 3 dargestellt.

Um Gewebe von Mäusen mit schlecht heilenden Wunden zu gewinnen, wurden BALB/c Mäuse vor der Verwundung mit Dexamethason (Injektion von 0,5 mg Dexamethason in isotonischer Salzlösung pro kg Körpergewicht zwei mal pro Tag für 5 Tage) behandelt. Um Gewebe von jungen und alten Mäusen zu gewinnen, wurden 1-Tageswunden von 4 Wochen alten und 12 Monate alten BALB/c Mäusen verwendet. Um Wundgewebe von Mäusen mit Diabetes zu erhalten, wurden 1 Tageswunden von 10 Wochen alten C57BL/Ks-*db/db*/Ola Mäusen verwendet. Als Kontrolltiere wurden in diesem Fall 10 Wochen alte C57BL/Ks Mäuse verwendet. Gesamt-RNA wurde wie oben beschrieben aus Haut und Wundgewebe gewonnen und 1 µg Gesamt-RNA wurde mit dem TaqMan Reverse Transcription Reagents Kit (Perkin Elmer) nach den Empfehlungen des Herstellers (SYBR Green PCR and RT-PCR Reagents-Protokol, Perkin Elmer Applied Biosystems, 1998) in einem Thermocycler (GeneAmp PCR-System 9700, PE) revers transkribiert. Die Primer fiir die Amplifikation der SW1368 cDNA (*SW1368*-Primer 1: GAGGCATGTCAAATCAGTAAGCTG (SEQ ID Nr. 9), *SW1368*-Primer 2: GGTGGCTTTGGAGTGAGCAC (SEQ ID Nr. 10) und der Referenz (GAPDH-Primer 1: ATCAACGGGAAGCCCATCA (SEQ ID Nr. 11), GAPDH-Primer 2: GACATACTCAGCACCGGCCT (SEQ ID Nr. 12)) wurden anhand der vorangehend beschriebenen cDNA Sequenz von SW1368 aus Maus und der bekannten Sequenz von GAPDH aus Maus mit der Primer-Express-Software für Macintosh PPC Version 1.0 (PE Applied Biosystems, P/N 402089, 1998) ausgewählt. Für die PCR wurde das SYBR Green PCR Core Reagents Kit (4304886, PE Applied Biosystems) verwendet. Die Konzentration der Primer in der PCR wurde zunächst im Bereich von 50 nM bis 600 nM optimiert und die Spezifität der PCR durch Analyse der Länge der amplifizierten Produkte in einer Agarose-Gel Elekrophorese geprüft. Anschließend wurde mittels einer Verdünnungsreihe die Effizienz der PCR-Systeme ermittelt (User Bulletin #2, Relative Quantitation of Gene Expression, PE Applied Biosystems, 1997). Dabei ergab sich, daß für beide cDNAs die Effizienz der Amplifikation bei 100% lag, d. h. bei jeder 1:2 Verdünnung der cDNA wurde ein Zyklus mehr benötigt, um den Fluoreszensschwellenwert zu überschreiten.

Für die Quantifizierung wurden je Ansatz cDNA aus 10 ng revers transkribierter Gesamt-RNA in einem Gesamtvolumen von 25 µl amplifiziert. Die Laufbedingungen für die PCR entsprachen den Angaben des Herstellers (PE Applied Biosystems, SYBR Green PCR and RT-PCR Reagents Protocol, 1998). Die CT-Werte wurden analysiert und daraus wurde die Häufigkeit von SW1368 mRNA relativ zu GAPDH mRNA berechnet. Dabei bestätigte sich sowohl die Zunahme von *SW1368* in normal heilenden Wunden im Vergleich zu intakter Haut von Kontrolltieren als auch in Wunden im Vergleich zu intakter Haut von jungen und alten Mäusen (Figur 2, vergleiche Figur 1). Die Analyse weiterer Wundheilungszustände ergab, daß die Expression von SW1368 in schlecht heilenden Wunden von mit Dexamethason behandelten Mäusen im Vergleich zu Wunden von Kontrolltieren leicht erhöht war. Dagegen konnte eine um 50% verringerte Menge an SW1368 in Wunden von Mäusen mit Diabetes im Vergleich zu Kontrollmäusen gezeigt werden (Figur 3). Somit konnte nicht nur die wundspezifische Regulation von SW1368 bestätigt werden, sondern es konnte zusätzlich eine Rolle bei gestörter Wundheilung nachgewiesen werden (Figur 3). Außerdem konnte gezeigt werden, daß das SW1695 wundspezifisch reguliert wird (Figur 2). In diesem Fall wurde in schlecht heilenden Wunden von mit Dexamethason-behandelten Tieren eine 3-fach höhere Menge an SW1695 gemessen als in Wunden von Kontrolltieren. Dies verdeutlicht, daß sowohl SW1368 als auch SW1695 in verschiedenen Wundheilungsprozessen reguliert werden.

### Beispiel 3: Analyse des Expressionsmusters von SW1695 in humanen Wunden

Von unbehandelter intakter Haut und von unbehandelten 1- und 5-Tages Wunden von gesunden Probanden wurden mittels Stanzen Hautproben mit 4 mm Durchmesser entnommen. Diese wurden mittels "real time quantitative RTPCR" wie im Beispiel 2 beschrieben, untersucht. Die Primer für die Amplifikation der SW1695 cDNA (*SW1695*-Primer 1: TTCTTCTGCTTTGTGGCAAGG (SEQ ID Nr. 13), *SW1695*-Primer 2: GAAAAGGATCAGGAAGACCGG (SEQ ID Nr. 14) und der Referenz (hGAPDH-Primer 1: CATGGGTGTGAACCATGAGAAG (SEQ ID Nr. 16), hGAPDH-Primer 2: CTAAGCAGTTGGTGGTGCAGG (SEQ ID Nr. 17)) wurden anhand der offengelegten cDNA Sequenz von humanem SW1695 und anhand der bekannten Sequenz von humanem GAPDH ausgewählt. Für die Quantifizierung wurden je Ansatz cDNA aus 10 ng revers transkribierter Gesamt-RNA in einem Gesamtvolumen von 25 µl amplifiziert. Die PCR wurde entsprechend den Angaben des Herstellers (PE Applied Biosystems, SYBR Green PCR and RT-PCR Reagents Protocol, 1998) durchgeführt. Die CT-Werte wurden analysiert und daraus wurde die Häufigkeit von SW1695 mRNA relativ zu GAPDH mRNA berechnet. Es wurde eine Abnahme von *SW1695* um 60% in 1-Tageswunden im Vergleich zu intakter Haut gemessen. Diese Meßwerte stimmen mit dem im Mausmodell erhaltenen Ergebnissen überein, bei denen eine vergleichbare Abnahme an murinem *SW1695* in normal heilenden Wunden im Vergleich zu intakter Haut von Kontrolltieren beobachtet wurde (Figur 2). Zudem konnte in humanen 5-Tageswunden eine um 70% verringerte *SW1695* Expression im Vergleich zu intakter Haut nachgewiesen werden. Dies verdeutlicht, daß die SW1695 Expression nicht nur kurzfristig reguliert wird, sondern daß eine veränderte Expression über einen längeren Zeitraum für den Heilungsprozeß essentiell ist.

### Beispiel 4: Wundheilungskinetik der SW1368 Expression im Menschen

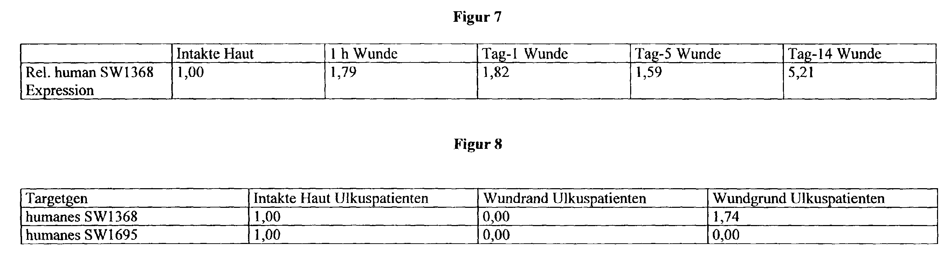

Um eine bessere zeitliche Auflösung des Expressionsverlauf eines erfindungsgemäßen GPCRs bei der Wundheilung zu erhalten, wurden von 6 Patienten 4 mm Biopsien von intakter Haut wie oben beschrieben entnommen, sowie 6 mm Biopsien zu den Zeitpunkten T=1 h, 1 d, 5 d und 14 d. Die Biopsien pro Zeitpunkt wurden gepoolt und die RNA isoliert. Die Isolierung der RNA erfolgte durch Homogenisieren der Biopsien in RNAclean Puffer (AGS, Heidelberg), dem 1/100 Volumenanteil 2-Mercaptoethanol zugesetzt worden war unter Verwendung eines Dispersers. Anschließend wurde die RNA durch zweimaliges Phenolisieren mittels mit Wasser gesättigtem saurem Phenol und in Gegenwart von 1-Brom-3-Chlor-Propan extrahiert. Anschließend wurden eine Isopropanol- und eine Ethanol-Fällung durchgeführt und die RNA mit 75% Ethanol gewaschen. Danach wurde ein DNase I-Verdau der RNA durchgeführt. Hierzu wurden 20 µg RNA (ad 50 µl mit DEPC-behandeltem Wasser) mit 5,7 µl Transkriptions-Puffer (Roche), 1 µl RNase-Inhibitor (Roche; 40 U/µl) und 1 µl DNase I (Roche; 10 U/µl) 20 min bei 37°C inkubiert. Dann wurde wiederum 1 µl DNase I zugegeben und weitere 20 min bei 37°C inkubiert. Anschließend wurde die RNA phenolisiert, Ethanolgefällt und gewaschen. Alle oben aufgeführten Schritte wurden mit DEPC (Diethylpyrocarbonat)-behandelten Lösungen bzw. Flüssigkeiten durchgeführt soweit diese keine reaktiven Aminogruppen enthielten. Anschließend erfolgte die Herstellung von cDNA aus der extrahierten RNA. Dies erfolgte in Gegenwart von 1 x TaqMan RT-Puffer (Applied Biosystems), 5,5 mM MgCl₂ (Perkin Elmer), je 500 µM dNTPs (Perkin Elmer), 2,5 µM Random Hexamere (Perkin Elmer), 1,25 U/µl MultiScribe Reverse Transcriptase (50 U/µl Perkin Elmer), 0,4 U/µl RNase Inhibitor (20 U/µl, Perkin Elmer), 20 µl RNA (50 ng/µl) und DEPC-behandeltem Wasser (ad 100 µl Volumen). Nach Zugabe der RNA und guter Durchmischung wurde die Lösung auf 2 0,2 ml Gefäße verteilt (je 50 µl) und die reverse Transkription in einem Thermocycler durchgeführt (10 min bei 25°C; 30 min bei 48°C und 5 min bei 95°C). Die nachfolgende Quantifizierung der cDNA erfolgte mittels quantitativer PCR unter Verwendung des SYBR Green PCR Master Mixes (Perkin Elmer), wobei jeweils eine Dreifachbestimmung (mit humanen SW1368-Primern und Cyclophilin-Primern) durchgeführt wurde. Die Stammlösung für jedes Triplett enthielt bei 57 µl Gesamtvolumen 37,5 µl 2 x SYBR Master Mix, 0,75 µl AmpErase UNG (1 U/µl) und 18,75 µl DEPC-behandeltes Wasser. Pro Dreifachbestimmung wurden zu 57 µl Stammlösung je 1,5 µl Vorwärts- und Rüchwärts-Primer (hSW1368-Primer 1: GGAGTCAGCC CTAAACTATT CCAG (SEQ ID Nr. 20); hSW1368-Primer 2: AGGTAGGCCG TGTGCACTGT (SEQ ID Nr. 21) in einem zuvor optimierten Konzentrationsverhältnis hinzugefügt. 60 µl der Stammlösung/Primer-Mischung wurde mit 15 µl cDNA-Lösung (2 ng/µl) gemischt und auf 3 Reaktionsgefäße verteilt. Parallel dazu wurde eine Stammlösung mit Primern zur Bestimmung von Cyclophilin (Cyclophilin-Primer 1: TCTTAACCAC CAGATCATTC CTTCT (Seq ID Nr. 18) und Cyclophilin-Primer 2: GGATACTGCG AGCAAATGGG (Seq ID Nr. 19)) als Referenz hergestellt, mit weiteren 15 µl der gleichen cDNA-Lösung gemischt und auf 3 Reaktionsgefäße verteilt. Außerdem wurden um eine Standardkurve für die Cyclophilin-PCR zu erstellen verschiedene cDNA-Lösungen als Verdünnungsreihe hergestellt (4 ng/µl; 2 ng/µl; 1 ng/µl; 0,5 ng/µl und 0,25 ng/µl). Je 15 µl dieser cDNA-Löungen wurden mit 60 µl Stammlösung/Primer-Mischung zur Bestimmung von Cyclophilin gemischt und auf 3 Reaktionsgefäße verteilt. Ebenso wurde jeweils eine Standardkurve für die PCR des zu untersuchenden humanen SW1368 Gens erstellt; dabei wurden dieselben Verdünnungen, die auch für die Cyclophilin-Standardkurve zum Einsatz kamen, verwendet. Als Kontrolle diente ein PCR Ansatz ohne cDNA. Zu jeweils 60 µl Stammlösung/Primer-Mischung von jeweils humanem SW1368 und Cyclophilin wurden je 15 µl DEPC-Wasser gegeben, gemischt und jeweils auf 3 Reaktionsgefäße verteilt. Die Amplifikation der Ansätze wurde im GeneAmp 5700 durchgeführt (2 min bei 50°C; 10 min bei 95°C, gefolgt von 3 Zyklen mit 15 sek bei 96°C und 2 min bei 60°C; danach 37 Zyklen mit 15 sek bei 95°C und 1 min bei 60°C). Die Auswertung erfolgte durch die Bestimmung der relativen Abundanz des humanem SW1368-Gens im Bezug auf die Cyclophilin-Referenz. Dazu wurde zuerst eine Standardkurve erstellt, indem die C_{T}-Werte der Verdünnungsreihe gegen den Logarithmus der cDNA-Menge im PCR-Ansatz (in ng umgeschriebener RNA) aufgetragen wurden und die Steigung (s) der Geraden ermittelt wurde. Die Effizienz (E) der PCR ergibt sich dann wie folgt: E = 10^{-1/s} - 1. Die relative Abundanz (X) des SW1368 Gens (Y) im Bezug auf Cyclophilin (cyc) ist dann: X=(1+E_{cyc})^{C}_{T}^{(cyc)}/(1+E_{Y})^{C}_{T}^{(Y)}. Anschließend wurden die Zahlenwerte normiert, indem die Menge an SW1368 cDNA aus intakter Haut gleich 1 gesetzt wurde. Die Ergebnisse sind in Figur 7 zusammengestellt. Es wurde festgestellt, daß auch im Menschen eine, zwar im Vergleich zur Maus deutlich schwächere, jedoch signifikante Hochregulation der SW1368 Expression während der Wundheilung festzustellen ist, die am Tag-14 nach Wundsetzung am ausgeprägtesten ist. Dies verdeutlicht wiederum, daß die Regulation der erfindungsgemäßen GPCRs essentiell für die Wundheilung sind.

### Beispiel 5. Fehlregulation der SW1368 und SW1695 Expression in humanen Ulzera

Um zu zeigen, daß die als wundrelevant identifizierten SW1368 und SW1695 Gene nicht nur bei normal verlaufender Wundheilung sondern auch bei einer Wundheilungsstörung eine Rolle spielen, wurden Biopsien von Patienten mit chronischen venösen Ulzera (Ulcera venosa) gleichzeitig sowohl von intakter Haut als auch vom Wundgrund und Wundrand entnommen und auf Expression der Targetgene hin untersucht. Von jeder Gruppe (intakte Haut, Wundrand, Wundgrund) wurden die Biopsien von jeweils 6 Probanden gepoolt. Aus allen Biopsien wurde, wie im Beispiel 4 beschrieben, RNA isoliert und cDNA hergestellt. Die Quantifizierung wundheilungsrelevanter cDNAs erfolgte ebenfalls wie in Beispiel 4 beschrieben, wobei wiederum die Menge an Cyclophilin mRNA zur Berechnung der relativen Menge an Targetgen cDNA herangezogen wurde. Die Ergebnisse der Experimente sind in Figur 8 zusammengestellt. Es wurde bei beiden Genen eine Fehlregulation der Expression im Ulkus im Vergleich zur normal heilenden Wunde (vergleiche Figur 7 und Beispiel 3) festgestellt: während im Falle von SW1695 eine signifikante, jedoch vergleichsweise geringe Verminderung der Expression um 60-70% zum Tag-1 und Tag-5 nach Verwundung in normal heilenden Wunden beobachtet wurde, konnte weder im Wundgrund noch im Wundrand SW1695 mRNA nachgewiesen werden. Dies verdeutlicht, daß eine Fehlregulation der SW1695 Expression, insbesondere das Fehlen der mRNA zu schweren Wundheilungsstörungen führen kann. Im Falle von SW1368 konnte ebenfalls ein Fehlen der mRNA im Wundrand venöser Ulzera nachgewiesen werden, während bei normal verlaufender Wundheilung eine Hochregulation der SW1368 gezeigt wurde. Im Unterschied zu SW1695 konnte jedoch eine signifikante Menge an SW1368 mRNA im Wundgrund gemessen werden.Das absolute Fehlen beider erfindungsgemäßen GPCRs im Wundrand der Ulzera, der dem hyperproliferativen Epithel normal heilender Wunden entspricht zeigt daß beide Gene bei essentiellen Prozessen bei der Wundheilung, möglicherweise bei der Reepithalisierung durch die proliferierenden Keratinozyten des hyperproliferativen Epithels beteiligt sind. Dies zeigt, daß die Aktivität und/oder Expression der erfindungsgemäßen GPCRs bei der Behandlung der Wundheilung, insbesondere deren pathologischen Störungen, verändert werden muß, bevorzugterweise erhöht werden muß. Bevorzugterweise ist die Aktivität und/oder Expression lokal in der Haut und/oder Wunde zu erhöhen.

### Beispiel 7: Differentielle Regulation von humanem SW1695 in der Haut von Psoriasispatienten

Es sollte nun anhand von Psoriasis Patienten verifiziert werden, daß erfindungsgemäßen GPCRs nicht nur bei der Wundheilung und Wundheilungsstörungen eine wichtige Rolle spielen sondern auch bei anderen Hauterkrankungen, insbesondere Psoriasis. Dazu wurden Psoriasis Patienten 4 mm Stanzbiopsien sowohl von läsionaler als auch nicht-läsionaler Haut wie in Beispiel 4 beschrieben entnommen. Als Kontrolle wurde gesunden Probanden Biopsien intakter Haut entnommen. Die Isolierung der mRNA aus den einzelnen Biopsien erfolgte durch Einbettung der Biopsien in tissue freezing medium (Jung), das Zerkleinern der Biopsie unter Verwendung eines Mikrotoms und die darauffolgende mRNA Isolierung mittels Dynabeads-Oligo dT (Dynal). Die zerkleinerten Biopsien werden zunächst in Lyse-Puffer suspendiert und dann mit dem Polytron homogenisiert. Um die genomische DNA zu zerkleinern wird die Lösung über Qia-Shredder Columns abzentrifugiert und zusätzlich in einer Spritze mit Kanüle mehrmals geschert. Die Dynabeads wurden gemäß den Angaben des Herstellers vorbehandelt und (250 µl der Stammsuspension) mit dem Lyse-Homogenisat gemischt, inkubiert und gewaschen (Endvolumen 250 µl). Die Suspension wurde nun in je eine Portion von 240 µl sowie von 10 µl (als Kontrolle) geteilt. Für die Erststrangsynthese wurden folgende Komponenten gemischt: 20 µl 10x TaqMan RT Puffer, 44 µl 25 mM MgCl₂, 40 µl dNTP-Mix (2,5 mM/dNTP), 87 µl DEPC-H₂O, 4 µl RNase Inhibitor (20 U/µl) und 5 µl MultiScribe Transcriptase (50 U/µl). 195 µl des Reaktionsmixes wurden nun zum 240 µl Ansatz und 20 µl zum Kontrollansatz gegeben, gemischt und 45 min bei 48°C inkubiert. Die Dynabeads wurden anschließend im Magnetständer pelletiert und der Überstand abgenommen. Anschließend wurden 20 µl Tris-HCl Puffer zugegeben und die Suspension 1 min bei 95°C inkubiert. Die Dynabeads wurden sofort im Magnetständer pelletiert und die mRNA im Überstand abgenommen. Die cDNA/Dynabeads wurden anschließend 3x mit TE Puffer gewaschen. Für die Zweitstrangsynthese wurden die cDNA/Dynabeads 2x in 1x EcoPol Puffer gewaschen und eine Lösung folgender Komponenten zugegeben: 23 µl 10x EcoPol Puffer; 4,6 µl dNTP Mix (25 mM/dNTP); 11,5 µl Random Hexamers; 118,7 µl DEPC-H₂O. Die Suspension wurde kurz mit Hilfe des Vortexers gemischt und anschließend 9,2 µl Klenow Fragment (5 U/µl) zugegeben. Zum Ansatz wurden 200 µl dieser Lösung zugegeben, zum Kontrollansatz 20 µl, und die Suspensionen wurden bei 37°C 1 h inkubiert. Die DNA wurde dann bei 94°C 1 min aufgeschmolzen und die Dynabeads im Magnetständer pelletiert. Der Überstand wurde in ein neues Reaktionsgefäß transferiert und das Enzym bei 75°C 10 min inaktiviert. Die im Überstand enthaltene sense DNA-Stränge wurden dann für die Taq Man Analyse eingesetzt.

Die TaqMan Analyse wurde wie in Beispiel 4 beschrieben durchgeführt, wobei die Menge an GAPDH (siehe Beispiel 3) zur Berechnung der relativen Abundanz der SW1695 mRNA in den einzelnen Biopsien herangezogen wurde. Da aus den Hautbiopsien von Psoriasis Patienten, insbesondere aus läsionaler Haut eine weitaus größere Gesamt mRNA Menge isolierbar ist als aus intakter Haut gesunder Probanden, ist eine Normalisierung auf gleiche mRNA Mengen notwendig, wobei die Menge an GAPDH mRNA als Housekeeping Gen als Marker für die Gesamt mRNA-Menge angenommen wurde.
Insgsamt wurden 2 Biopsien von intakter Haut gesunder Probanden analysiert, sowie jeweils 4 Biopsien von läsionaler und nichtläsionaler Haut von Psoriasispatienten. Die Abundanzen der einzelnen Gruppen (intakte Haut, läsionale Haut, nichtläsionale Haut) wurden dann auf die Gesamtzahl der Abundanzen der auf einer Mikrotiterplatte gemessenen cDNAs normiert. Anschließend wurde der Mittelwert der normierten SW1695 Abundanzen für jede Gruppe (intakte Haut gesunder Probanden, läsionale bzw. nicht-läsionale Haut Psoriasispatienten) bestimmt und der Wert für die intakte Haut gesunder Probanden gleich 1,00 gesetzt. Die relative Menge an SW1695 in nicht-läsionaler Haut der Psoriasispatienten konnte ergab dann 3,80, während die läsionale Haut der Psoriasipatienten ein Wert von 1,11 ermittelt wurde. Dies zeigt, daß erfindungsgemäße GPCRs, insbesondere SW1695, als diagnostischer Marker für eine Psoriasis Prädisposition eingesetzt werden kann, da nicht-läsionale Haut eine erhöhte Menge an SW1695 mRNA gegenüber Haut gesunder Probanden aufweist. Weiterhin ist die Menge an mRNA in läsionaler Haut gegenüber nicht-läsionaler Haut verringert. Zusammenfassend beweisen die Ergebnisse dieses Versuches, daß die Fehlregulation der erfindungsgemäßen Gene und Polypetide, insbesondere SW1695, zu Hauterkrankungen, insbesondere Psoriasis rühren kann. Zur Prävention und/oder Behandlung von Hauterkrankungen, iinsbesondere Psoriasis, ist die Aktivität und/oder Expression der erfindungsgemäßen GPCRs, insbesondere SW1695, zu modulieren. Bevorzugterweise ist die Expression und/oder Aktivität zur Prävention läsionaler Psoriasishaut zu erniedrigen und ist bevorzugterweise zu erhöhen bei der Behandlung läsionaler psoriatischer Hautpartien.

Dies beweist somit zusammenfassend, daß die differentielle Expression von erfindungsgemäßen GPCRs nicht nur für die Wundheilung essentiell ist, sondern daß Fehlregulationen zu schweren Wundheilungsstörungen führen können und daß daher die erfindungsgemäßen GPCRs zur Diagnose, Prävention und/oder Behandlung von Erkrankungen, beispielsweise Hauterkrankungen, insbesondere Psoriasis, und/oder zur Diagnose und/oder Behandlung bei der Wundheilung und/oder deren krankhaften Störungen verwendet werden können.

## Patentansprüche

1. G-Protein-gekoppeltes Rezeptor-Polypeptid nach einer der SEQ ID Nr. 1 bis SEQ ID Nr. 3 oder funktionelle Varianten davon.

2. Polypeptid nach Anspruch 1, **dadurch gekennzeichnet, daß** das Polypeptid ein Fusionsprotein ist.

3. Nukleinsäure, die für ein Polypeptid nach Anspruch 1 oder 2 kodiert oder Varianten der Nukleinsäure.

4. Nukleinsäure nach Anspruch 3, **dadurch gekennzeichnet, daß** die Nukleinsäure in einem knock-out Genkonstrukt oder einer Expressionskassette oder in einem Vektor, ausgewählt aus Plasmiden, Shuttle Vektoren, Phagemiden, Cosmiden, Expressionsvektoren oder gentherapeutisch wirksamen Vektoren, enthalten ist.

5. Zelle, enthaltend eine Nukleinsäure nach Anspruch 3 oder transformiert mit einem Vektor, einem knock-out Genkonstrukt oder einer Expressionskassette gemäß Anspruch 4.

6. Zelle nach Anspruch 5, **dadurch gekennzeichnet, daß** es sich bei der Zelle um eine heterologe oder eine autologe Zelle, insbesondere um eine Hautzelle, eine Keratinozyte, eine Fibroblaste oder eine Endothelzelle handelt.

7. Transgene embryonale nichtmenschliche Stammzelle, **dadurch gekennzeichnet, daß** die Stammzelle eine Nukleinsäure nach Anspruch 3, oder einen Vektor, ein knock-out Genkonstrukt oder eine Expressionskassette nach Anspruch 4, enthält.

8. Transgenes nichtmenschliches Säugetier, **dadurch gekennzeichnet, daß** sein Genom eine Nukleinsäure nach einem der Ansprüche 3, oder einen Vektor, ein knock-out Genkonstrukt oder eine Expressionskassette nach Anspruch 4 enthält.

9. Antikörper oder Antikörperfragment, **dadurch gekennzeichnet, daß** der Antikörper oder das Antikörperfragment gegen ein Polypeptid gemäß einem der Ansprüche 1 oder 2 oder gegen eine Nukleinsäure gemäß einem der Ansprüche 3 oder 4 gerichtet ist.

10. Test zur Identifizierung von pharmakologisch aktiven Substanzen, **dadurch gekennzeichnet, daß** der Test mindestens ein Polypeptid nach einem der Ansprüche 1 oder 2, und/oder mindestens eine Nukleinsäure nach einem der Ansprüche 3 oder 4, und/oder mindestens einen Antikörper oder Antikörperfragment nach Anspruch 9, und/oder mindestens eine Zelle nach einem der Ansprüche 5 bis 7, und/oder ein transgenes nichtmenschliches Säugetier nach Anspruch 8, enthält, gegebenenfalls zusammen oder kombiniert mit geeigneten Zusatz- und/oder Hilfsstoffen.

11. Test gemäß Anspruch 10, **dadurch gekennzeichnet, daß** mindestens ein Polypeptid nach einem der Ansprüche 1 oder 2, und/oder mindestens eine Nukleinsäure nach einem der Ansprüche 3 oder 4, und/oder mindestens eine Zelle nach einem der Ansprüche 5 bis 7, und/oder mindestens einen Antikörper oder Antikörperfragment nach Anspruch 9, an eine Festphase gebunden wird.

12. Auf einem Trägermaterial fixiertes Array, **dadurch gekennzeichnet, daß** das Array mindestens ein Polypeptid nach einem der Ansprüche 1 oder 2, und/oder mindestens eine Nukleinsäure nach einem der Ansprüche 3 oder 4, und/oder mindestens eine Zelle nach einem der Ansprüche 5 bis 7, und/oder mindestens einen Antikörper oder Antikörperfragment nach Anspruch 9, enthält.

13. Diagnostikum, **dadurch gekennzeichnet, daß** das Diagnostikum mindestens ein Polypeptid gemäß Anspruch 1 oder 2, und/oder mindestens eine Nukleinsäure nach einem der Ansprüche 3 oder 4, und/oder mindestens eine Zelle nach einem der Ansprüche 5 bis 7, und/oder mindestens einen Antikörper oder Antikörperfragment nach Anspruch 9, enthält, gegebenenfalls zusammen oder kombiniert mit geeigneten Zusatz- und/oder Hilfsstoffen.

14. Diagnostikum nach Anspruch 13, **dadurch gekennzeichnet, daß** es eine Sonde, vorzugsweise eine DNA-Sonde, enthält.

15. Arzneimittel, **dadurch gekennzeichnet, daß** das Arzneimittel mindestens ein Polypeptid nach einem der Ansprüche 1 oder 2, und/oder mindestens eine Nukleinsäure nach einem der Ansprüche 3 oder 4, und/oder mindestens eine Zelle nach einem der Ansprüche 5 bis 7, und/oder mindestens einen Antikörper oder Antikörperfragment nach Anspruch 9, enthält, gegebenenfalls zusammen oder kombiniert mit geeigneten Zusatz- und/oder Hilfsstoffen.

16. Verwendung mindestens eines Polypeptids gemäß Anspruch 1 oder 2, und/oder mindestens einer Nukleinsäure nach einem der Ansprüche 3 oder 4, und/oder mindestens einer Zelle nach einem der Ansprüche 5 bis 7, und/oder mindestens eines Antikörpers oder Antikörperfragments nach Anspruch 9, zur Herstellung eines pharmazeutischen Präparates zur Diagnose, Prävention und/oder Behandlung von Hauterkrankungen, und/oder zur Diagnose und/oder Behandlung bei der Wundheilung und/oder deren krankhaften Störungen.

17. Verfahren zur Herstellung eines Polypeptids nach Anspruch 1 oder 2, **dadurch gekennzeichnet, daß** eine Nukleinsäure gemäß einem der Ansprüche 3 oder 4 in einer geeigneten Zelle exprimiert und gegebenenfalls isoliert wird.

18. Verfahren zur Herstellung eines transgenen nichtmenschlichen Säugetiers nach Anspruch 8, **dadurch gekennzeichnet, daß** eine transgene embryonale nichtmenschliche Stammzelle nach Anspruch 7 zu einem transgenen nichtmenschlichen Säugetier regeneriert wird.

19. Verfahren zur Herstellung eines Antikörpers oder Antikörperfragments nach Anspruch 12, vorzugsweise eines polyklonalen oder monoklonalen Antikörpers oder Antikörperfragments, **dadurch gekennzeichnet, daß** ein Antikörper produzierender Organismus mit einem Polypeptid nach einem der Ansprüche 1 oder 2 oder mit einer Nukleinsäure nach Anspruch 3 oder 4 immunisiert wird.

20. Verfahren zur Herstellung eines Diagnostikums nach einem der Ansprüche 13 oder 14, und/oder eines Arzneimittels nach Anspruch 15, und/oder eines Tests nach Anspruch 11, **dadurch gekennzeichnet, daß** mindestens ein Polypeptid nach einem der Ansprüche 1 oder 2, und/oder mindestens eine Nukleinsäure gemäß mindestens einem der Ansprüche 3 und/oder 4, und/oder mindestens einen Antikörper oder Antikörperfragment nach Anspruch 9, und/oder mindestens eine Zelle nach einem der Ansprüche 5 bis 7, mit geeigneten Zusatz- und/oder Hilfsstoffen kombiniert wird.

21. Verfahren zur Herstellung eines auf einem Trägermaterial fixierten Arrays, **dadurch gekennzeichnet, daß** mindestens ein Polypeptid nach einem der Ansprüche 1 oder 2, und/oder mindestens eine Nukleinsäure nach einem der Ansprüche 3 oder 4, und/oder mindestens eine Zelle nach einem der Ansprüche 5 bis 7, und/oder mindestens einen Antikörper oder Antikörperfragment nach Anspruch 9, auf einem Trägermaterial fixiert wird.

22. Verwendung eines Tests nach einem der Ansprüche 10 oder 11 zum Auffinden von pharmakologisch aktiven Substanzen in Verbindung mit Erkrankungen, insbesondere von Hauterkrankungen, Wundheilung und/oder deren krankhaften Störungen.

23. Verwendung eines Arrays nach Anspruch 12 zur Analyse von Erkrankungen, insbesondere von Hauterkrankungen, Wundheilung und/oder deren krankhaften Störungen.

24. Verwendung eines G-Protein-gekoppelten Rezeptor-Polypeptids nach der SEQ ID Nr. 4 oder einer funktionellen Variante davon, und/oder einer das Polypeptid oder seine funktionelle Variante kodierenden Nukleinsäure oder einer Variante davon, und/oder einer Zelle, die das Polypeptid oder eine funktionelle Variante davon oder eine das Polypeptid oder seine funktionelle Variante kodierende Nukleinsäure oder eine Variante davon exprimiert, und/oder eines gegen das Polypeptid oder seine funktionelle Variante gerichteten Antikörpers oder Antikörperfragments, und/oder des Polypeptids oder einer funktionellen Variante in Form eines Fusionsproteins, gegebenenfalls kombiniert oder zusammen mit geeigneten Zusatz- und/oder Hilfsstoffen, zur Herstellung eines pharmazeutischen Präparates zur Diagnose, Prävention und/oder Behandlung von Hauterkrankungen, und/oder zur Diagnose und/oder Behandlung bei der Wundheilung und/oder deren krankhaften Störungen.

25. Verwendung eines G-Protein-gekoppelten Rezeptor-Polypeptids nach der SEQ ID Nr. 4 oder einer funktionellen Variante davon, und/oder einer das Polypeptid oder seine funktionelle Variante kodierenden Nukleinsäure oder einer Variante davon, und/oder einer Zelle, die das Polypeptid oder seine funktionelle Variante oder eine das Polypeptid oder seine funktionelle Variante kodierende Nukleinsäure oder eine Variante davon exprimiert, und/oder eines gegen das Polypeptid oder seine funktionelle Variante gerichteten Antikörpers oder Antikörperfragments, und/oder des Polypeptids oder einer funktionelle Variante in Form eines Fusionsproteins, zur Identifizierung von pharmakologisch aktiven Substanzen in Verbindung mit Hauterkrankungen, Wundheilung und/oder deren krankhaften Störungen.

26. Verwendung eines G-Protein-gekoppelten Rezeptor-Polypeptids nach der SEQ ID Nr. 4 oder einer funktionellen Variante davon, und/oder einer das Polypeptid oder seine funktionelle Variante kodierenden Nukleinsäure oder einer Variante davon, und/oder einer Zelle, die das Polypeptid oder seine funktionelle Variante oder eine das Polypeptid oder seine funktionelle Variante kodierende Nukleinsäure oder eine Variante davon exprimiert, und/oder eines gegen das Polypeptid oder seine funktionelle Variante gerichteten Antikörpers oder Antikörperfragments, und/oder des Polypeptids oder einer funktionellen Variante in Form eines Fusionsproteins, zur Herstellung eines Tests zur Auffindung von pharmakologisch aktiven Substanzen in Verbindung mit Hauterkrankungen, Wundheilung und/oder deren krankhaften Störungen.

27. Verwendung nach Anspruch 26, **dadurch gekennzeichnet, daß** zur Herstellung eines Tests mindestens ein Polypeptid nach Anspruch 26, und/oder mindestens ein Fusionsprotein nach Anspruch 26, und/oder mindestens eine Nukleinsäure nach Anspruch 26, und/oder mindestens ein Antikörper oder Antikörperfragment nach Anspruch 26, und/oder mindestens eine Zelle nach Anspruch 26, an eine Festphase gebunden wird.

28. Verwendung mindestens eines G-Protein-gekoppelten Rezeptor-Polypeptids nach der SEQ ID Nr. 4 oder einer funktioneller Variante davon, und/oder mindestens einer das Polypeptid oder seine funktionelle Variante kodierenden Nukleinsäure oder einer Variante davon, und/oder mindestens eines gegen das Polypeptid oder seine funktionelle Variante gerichteten Antikörpers oder Antikörperfragments, und/oder mindestens das Polypeptids oder einer funktionelle Variante in Form eines Fusionsproteins, und/oder mindestens einer Zelle, die das Polypeptid oder seine funktionelle Variante oder eine das Polypeptid oder seine funktionelle Variante kodierende Nukleinsäure oder eine Variante davon exprimiert, zur Herstellung eines auf einem Trägermaterial fixierten Arrays zur Analyse von Hauterkrankungen, Wundheilung und/oder deren krankhaften Störungen.

29. Verwendung nach einem der Ansprüche 24 bis 28, **dadurch gekennzeichnet, daß** die Nukleinsäure in Form eines knock-out Genkonstrukt oder einer Expressionskassette oder in Form eines Vektors ausgewählt aus einer Gruppe von Plasmiden, Shuttle Vektoren, Phagemiden, Cosmiden, Expressionsvektoren oder gentherapeutisch wirksamen Vektoren, eingesetzt wird.

30. Verwendung nach einem der Ansprüche 24 bis 29, **dadurch gekennzeichnet, daß** es sich bei der Zelle um eine heterologe oder eine autologe Zelle, insbesondere um eine Hautzelle, eine Keratinozyte, eine Fibroblaste oder eine Endothelzelle handelt.

31. Verwendung nach mindestens einem der Ansprüche 16 und 22 bis 30, **dadurch gekennzeichnet, daß** die krankhafte Störung der Wundheilung ein Ulkus der Haut, vorzugsweise ein venöser Ulkus ist.

32. Verwendung nach mindestens einem der Ansprüche 16 und 22 bis 31 **dadurch gekennzeichnet, daß** die Hauterkrankung Psoriasis ist.
